# FASCICULE DE BREVET EUROPEEN

(11) **EP 3 349 585 B1**
(45) Date de publication et mention de la délivrance du brevet: **03.11.2021**
(21) Numéro de dépôt: 16767261.7
(22) Date de dépôt: 18.09.2016
(51) Int. Cl.: A01P 1/00, A01N 59/24, A01N 59/12

(54) **COMPOSITION COMPRENANT DES IONS I2SCN- ET/OU DES IONS I(SCN)2-**
ZUSAMENSETZUNGEN ENTHALTEND I2SCN- UND/ODER I(SCN)2- IONEN
COMPOSITION COMPRISING I2SCN- IONS AND/OR I(SCN)2- IONS

(30) Priorité: 17.09.2015 FR 1558780
(43) Date de publication de la demande: 25.07.2018
(73) Titulaire: Taradon Laboratory, 1480 Tubize (BE)
(72) Inventeur: BAFORT, Françoise, 5002 Saint-servais (BE); JIJAKLI, Mohamed Haïssam, 5361 Scy (BE); PERRAUDIN, Jean-Paul, 1180 Bruxelles (BE)
(74) Mandataire: Tripoz, Inès
(86) Numéro de dépôt international: PCT/EP2016/072088
(87) Numéro de publication internationale: WO 2017/046407

(56) Documents cités:
- EP-B1- 1 349 457
- WO-A1-00/01237
- WO-A1-2016/026946
- Lynn L Lewis ET AL: "PART A: A STUDY OF SOME THIOCYANATO COMPLEXES OF RHENIUM. PART B: THE GRAVIMETRIC AND SPECTROPHOTOMETRIC ANALYSIS OF MICRO AMOUNTS OF RHENIUM", Trans. Favaday SOL, 1 janvier 1955 (1955-01-01), pages 2191-875, XP055259088, Extrait de l'Internet: URL:http://pubs.acs.org/doi/pdf/10.1021/ja 00866a007 [extrait le 2016-03-17] cité dans la demande

## Description

La présente invention est relative à la production de composés antimicrobiens et à leurs utilisations, y compris en combinaison avec d'autres molécules pour la préparation de médicaments, pour la prophylaxie ou la thérapie des maladies infectieuses causées par des micro-organismes, pour la protection des plantes contre leurs agents pathogènes et leurs ravageurs, ou encore pour l'amélioration de la qualité de certains produits, comme par exemple certaines peintures.

Plus précisément, elle concerne le domaine des composés antimicrobiens produits par oxydation d'ion thiocyanate en présence d'un halogène, ladite oxydation étant catalysée à l'aide d'enzymes particulières appelées oxydoréductases, et plus précisément des peroxydases, la préférée étant la lactoperoxydase (LP).

Dans le cadre de la présente demande, le terme « halogène » désigne, parmi les éléments chimiques de la 17^{ème} colonne du tableau périodique (anciennement appelé groupe VII ou VIIA), particulièrement le chlore (Cl), le brome (Br) et l'iode (I).

Les halogènes peuvent donner lieu à la formation d'ions de type « X⁻ », appelés « ions halogénures » : ion chlorure (Cl⁻), ion bromure (Br⁻) et ion iodure (I⁻).

De manière générale, le terme « pseudo-halogène » désigne des composés binaires inorganiques de la forme « MN », dans laquelle :
- M est un cyanure (CN⁻), cyanate (OCN⁻) ou thiocyanate (SCN⁻) ; et
- N est un de ces mêmes groupes ou un vrai halogène tel que défini plus haut.

Dans le cadre de la présente demande, il sera entendu que le terme « pseudo-halogène » désigne préférentiellement les ions thiocyanate (SCN⁻).

Dans le cadre de la présente demande, le terme « interhalogène » désigne des composés formés de plusieurs halogènes tels que définis plus haut, identiques ou différents. A titre d'exemple, nous pouvons citer l'ion triodure I₃⁻ (halogènes identiques), ou encore le monochlorure d'iode de formule ICI (halogènes différents).

Dans le cadre de la présente demande, le terme « interpseudohalogène » désigne des composés formés d'au moins un halogène tel que définis plus haut et de thiocyanate (SCN⁻). A titre d'exemple, nous pouvons citer les espèces suivantes : I₂SCN⁻, ISCN et I(SCN)₂⁻.

De manière générale, il est connu que les halogénures (Cl⁻, Br⁻ ou I⁻) ou les pseudohalogénures (SCN⁻) peuvent être oxydés, par exemple en présence de peroxyde d'hydrogène (H₂O₂) (ou d'un système donneur de H₂O₂).

Les équations des réactions sont :

H₂O₂ + X⁻ (Cl⁻, Br⁻ ou I⁻) → OX⁻ + H₂O

H₂O₂ + SCN⁻ → OSCN⁻ + H₂O

Ces réactions d'oxydation peuvent également être réalisées en présence d'enzymes particulières.

Ces réactions d'oxydations peuvent également être réalisées (avec ou sans enzyme(s)) en présence à la fois d'un ion halogénure (X⁻) et de SCN⁻. Ces réactions d'oxydations peuvent également être réalisées en présence d'enzymes particulières.

En biochimie, les enzymes de la classe des oxydoréductases sont notamment classées en différents groupes : oxydases, réductases, peroxydases, oxygénases, hydrogénases, déshydrogénases, etc.

Plus particulièrement, les peroxydases sont des enzymes très répandues dans le monde vivant. Dans l'organisme, elles décomposent notamment les composés peroxydes toxiques.

En laboratoire, les peroxydases sont très utilisées, par exemple la peroxydase de raifort (HRP) est notamment largement utilisée en biotechnologie comme réactif de détection dans les immuno-essais.

Dans le groupe des peroxydases, on distingue notamment les peroxydases à hème.

Les peroxydases à hème sont présentes chez les plantes et les mammifères.

Leur rôle chez les plantes est multiple : métabolisme de l'auxine, défense extracellulaire, biosynthèse et dégradation de la lignine, dégradation du peroxyde d'hydrogène et oxydation de réducteurs toxiques. Les peroxydases des plantes sont induites par le stress par exemple suite à une attaque de pathogènes, des blessures, la chaleur, le froid, la sécheresse ou la lumière UV.

Les peroxydases de mammifères quant à elles jouent un rôle dans la production d'hormone thyroïdienne, dans la détoxification du peroxyde d'hydrogène et également comme système de défense naturel contre les pathogènes.

On retrouve dans le groupe des peroxydases notamment la lactoperoxydase (LP), la peroxydase thyroïdienne (TPO), la myéloperoxydase (MPO), la peroxydase salivaire (SPO) et la peroxydase éosinophile (EPO).

En présence de substrats spécifiques, elles vont catalyser une réaction d'oxydation et générer des espèces oxydantes, responsables par exemple de l'activité antimicrobienne. La spécificité des substrats est caractéristique du type de peroxydase.

La lactoperoxydase (LP) est présente dans le lait de vache à des concentrations d'environ 30 mg/L, concentration qui varie selon la saison, l'alimentation de la vache mais surtout le stade de lactation (concentration maximale 3 à 5 jours après le vêlage).

L'une de ses fonctions biologiques réside en un effet bactériostatique/bactéricide en présence de peroxyde d'hydrogène (H₂O₂) et de thiocyanate (SCN⁻). La lactoperoxydase (LP) peut également oxyder certains halogénures, par exemple l'ion iodure (I).

Les réactions d'oxydation catalysée par la lactoperoxydase (LP) peuvent se résumer comme suit :

H₂O₂ + X⁻ (Cl⁻, Br⁻ ou I⁻) + LP → OX⁻ + H₂O + LP

H₂O₂ + SCN⁻ + LP → OSCN⁻ + H₂O + LP

Dans l'art antérieur, il est connu que les espèces *hypoiodite* (OI⁻) et *hypothiocyanite* (OSCN⁻) ont un effet bactériostatique. Ces espèces vont réagir, par exemple, avec les groupements NH₂ ou thiols (-SH) d'enzymes essentielles au métabolisme du pathogène.

Dans l'art antérieur, certaines tentatives de mélange des ions I⁻ et SCN⁻ préalablement à la mise en contact avec le peroxyde d'hydrogène et la lactoperoxydase (LP) ont été réalisées.

On connait le produit vendu sous la dénomination KiB500®, qui inclut de la lactoperoxydase (LP) et des ions thiocyanates. Ce produit est actif contre certaines bactéries et virus. L'activité antimicrobienne est due aux ions hypothiocyanite (OSCN).

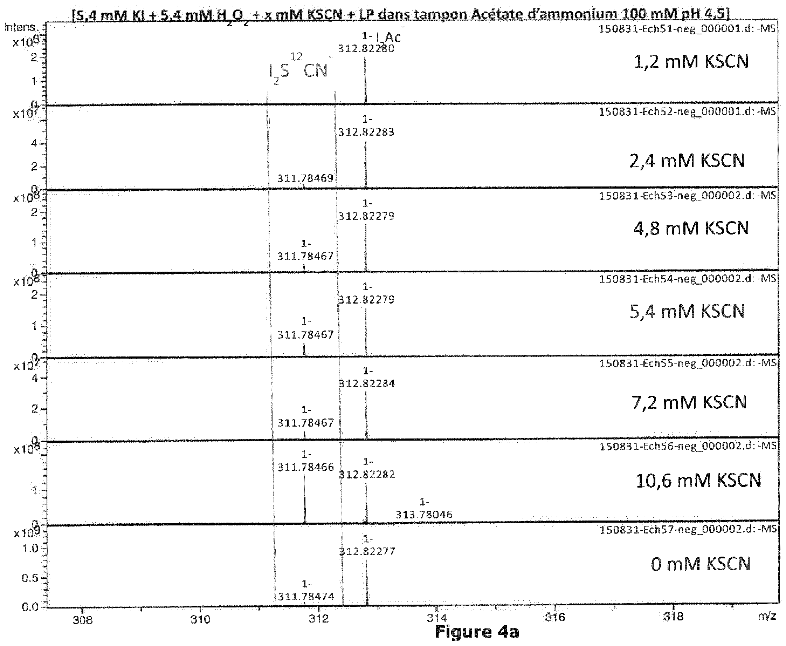

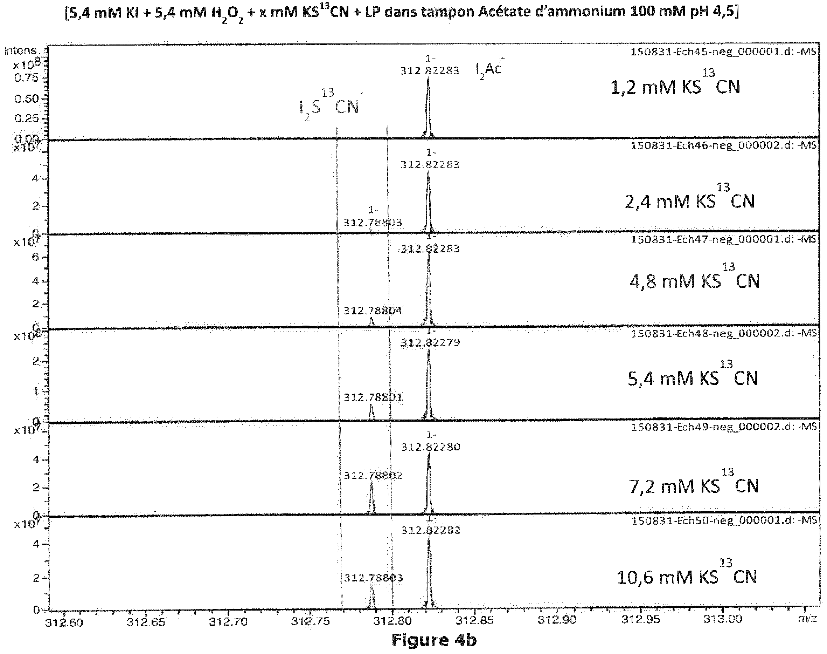

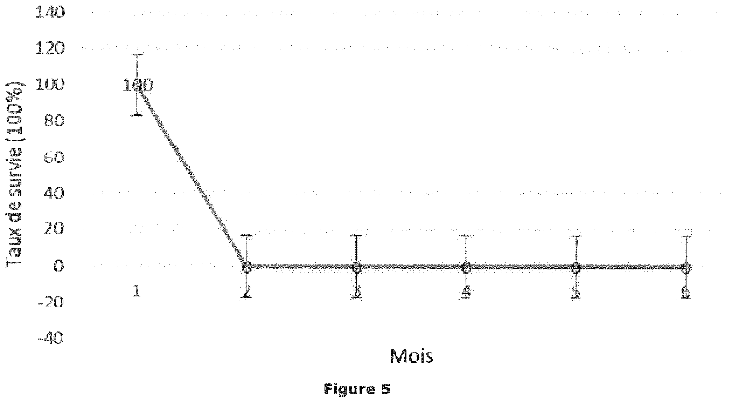

Dans la publication Bosh et al. "The lactoperoxydase system : the influence of iodide and the chemical and antimicrobial stability over the period of about 18 months" (Journal of Applied Microbiology 2000, 89, 215-224), un système comprenant la lactoperoxydase (LP), des ions thiocyanates (SCN⁻) et des ions iodures (I⁻) est étudié quant à ses propriétés bactéricides et fongicides, ainsi que leur durée. Il est déductible des diagrammes de la page 217 que les ions iodure améliorent l'efficacité du système lactoperoxydase (LP). Il est également déductible des figures 4 et 5 que lorsque le système est conservé en présence d'oxygène, l'activité bactéricide diminue, notamment lorsque la composition est conservée plus de 200 jours. Les auteurs précisent que les ions formés sont des ions OI⁻ et OSCN⁻, connus pour leur activité bactéricide/bactériostatique. Il est précisé que ces ions ont une concentration restant stable pendant environ 1 mois. Les réactions d'oxydation ont lieu « en parallèle » entre les ions SCN⁻ et I⁻.

Dans la publication Bafort et al. "Mode of Action of Lactoperoxydase as Related to Its Antimicrobial Activity : A Review" (Hindawi Publishing Corporation Enzyme Research, Volume 2014, Article ID 517164, 13 pages), les espèces chimiques actives dérivées des ions thiocyanates ou des ions iodures par un système lactoperoxydase (LP) sont étudiées. Il est précisé en fin d'article que s'agissant de l'activité antimicrobienne d'un système lactoperoxydase-iodure-thiocyanate, des résultats contradictoires ont été obtenus selon la souche bactérienne.

Ainsi, dans les démarches de l'art antérieur, lorsque le système lactoperoxydase (LP) fonctionne avec SCN⁻ et I⁻, ces derniers entrent en compétition pour se fixer sur le site de fixation de la lactoperoxydase (LP) pour produire, en présence de peroxyde d'hydrogène (H₂O₂), conjointement OSCN⁻ et OI⁻.

Le schéma réactionnel décrit dans l'art antérieur est le suivant :

H₂O₂ + SCN⁻ + I⁻ + lactoperoxydase (LP) → H₂O + OSCN⁻ + OI⁻ + lactoperoxydase (LP).

De manière surprenante, il a été observé que l'oxydation conjointe d'ions I⁻ et SCN⁻ en présence de H₂O₂ et en présence, pendant une durée limitée, de lactoperoxydase (LP) et à un pH compris entre 4 et 8, la composition obtenue présentait une activité antimicrobienne fortement augmentée, c'est-à-dire supérieure aux compositions de l'art antérieur comprenant les ions OSCN⁻ et OI⁻.

Dans des conditions particulières, il a été démontré que d'autres espèces que les ions OSCN⁻ et OI⁻ étaient formées et de manière encore plus surprenante, il a été mis en évidence qu'il n'y avait même aucune formation d'ions OSCN⁻.

Il a été démontré que les espèces ainsi formées étaient non seulement très actives, mais de plus beaucoup plus stables que les compositions traditionnelles comprenant des ions OSCN⁻ et OI⁻.

La solution obtenue est exempte d'ions OSCN⁻ et comprend un mélange d'ions de type « interpseudohalogène » suivants : I₂SCN⁻ et I(SCN)₂⁻.

Dans l'art antérieur, ces deux espèces I₂SCN⁻ et I(SCN)₂⁻ ont été citées par Lewis, C. & Skoog, D.A., 1962. Spectrophotometric study of a thiocyanate complex of iodine, Journal of the American Chemical Society, 84(7), pp.1101-1106.

Des compositions obtenues par oxydation chimique d'un mélange halogénure/thiocyanate ont été décrites dans l'art antérieur notamment dans Lewis, C. & Skoog, D.A., 1962. Spectrophotometric study of a thiocyanate complex of iodine, Journal of the American Chemical Society, 84(7), pp.1101-1106 et dans WO2016/026946. Ces compositions de par la cinétique de formation extrèmement lente ne permettent pas d'obtenir des compositions comprenant comme espèce chimique prépondérante issue de l'oxydation d'un mélange halogénure thiocyanate un ion choisi dans le groupe constitué par les ions I₂SCN⁻ et/ou I(SCN)₂⁻.comme en atteste le spectre de masse annexé à WO2016/026946. Ainsi, comme démontré dans les exemples, l'activité antimicrobienne des compositions obtenues n'est pas comparable avec celle des compositions selon l'invention.

Des compositions obtenues par oxydation enzymatique d'un mélange halogénure/thiocyanate ont été décrites dans l'art antérieur notamment dans EP1349457 ou WO2016026946. Ces compositions comprennent une enzyme qui reste de manière permanente dans la composition. Comme il a été démontré dans les exemples la présence concomittante de l'enzyme et des ions I₂SCN⁻ et/ou I(SCN)₂⁻ pendant un temps supérieur à 60 minutes dégrade I₂SCN⁻ et/ou I(SCN)₂⁻ et provoque la disparition complète des ions I₂SCN⁻ et/ou I(SCN)₂⁻ après 48 h de présence conjointe.

L'invention concerne une composition stable comprenant au moins un ion choisi dans le groupe constitué des ions I₂SCN⁻ et des ions I(SCN)₂⁻, ladite composition étant exempte d'ions hypothiocyanite (OSCN⁻).

Ladite composition est obtenue par oxydation enzymatique d'un mélange halogénure thiocyanate.

Dans ladite composition l'espèce chimique prépondérante issue de l'oxydation d'un mélange halogénure thiocyanate est un ion choisi dans le groupe constitué par les ions I₂SCN⁻ et/ou I(SCN)₂⁻.

Dans un mode de réalisation, la composition stable selon l'invention est caractérisée en ce qu'elle comprend en association des ions I₂SCN⁻ et I(SCN)₂⁻, ladite composition étant exempte d'ions hypothiocyanite (OSCN⁻).

Dans un mode de réalisation, la composition stable selon l'invention est caractérisée en ce qu'elle comprend l'ion I₂SCN⁻.

un mode de réalisation, la composition stable selon l'invention est caractérisée en ce qu'elle comprend l'ion I(SCN)₂⁻.

Dans un mode de réalisation, la composition stable selon l'invention est caractérisée en ce qu'elle comprend en outre du thiocyanate d'iode ISCN.

Dans un mode de réalisation, la composition stable selon l'invention est caractérisée en ce qu'elle comprend en outre au moins un composé choisi dans le groupe constitué de la lactoferrine, du lysozyme, des immunoglobines, des facteurs de croissance et leurs mélanges.

Dans un mode de réalisation, la composition stable selon l'invention est caractérisée en ce qu'elle comprend en outre au moins un composé choisi dans le groupe constitué de la lactoferrine, du lysozyme, des immunoglobines, un ou plusieurs facteur(s) de croissance et leurs mélanges.

Dans un mode de réalisation, la composition stable selon l'invention est caractérisée en ce qu'elle comprend en outre au moins un composé choisi dans le groupe constitué de la lactoferrine, du lysozyme, des immunoglobines, un ou plusieurs facteur(s) de croissance et leurs mélanges, caractérisé en ce que au moins un facteur de croissance est choisi dans le groupe constitué du Platelet Derived Growth Factor (PDGF), du Fibroblast Growth Factor (FGF), du Transforming Growth Factor (TGF), de l'angiogénine, de l'Epidermal Growth Factor (EGF), ou un mélange de ceux-ci.

Dans un mode de réalisation, la composition stable selon l'invention est caractérisée en ce qu'elle comprend en outre au moins un composé choisi dans le groupe constitué de la lactoferrine, du lysozyme, des immunoglobines, un ou plusieurs facteur(s) de croissance et leurs mélanges, caractérisé en ce que au moins un facteur de croissance est apporté par une source d'approvisionnement, ladite source d'approvisionnement étant du lactosérum de colostrum écrémé ou non ou du colostrum écrémé ou non.

Dans un mode de réalisation, la composition stable selon l'invention est caractérisée en ce qu'elle comprend en outre au moins de la lactoferrine.

Dans un mode de réalisation, la composition stable selon l'invention est caractérisée en ce qu'elle comprend en outre au moins du lysozyme.

Dans un mode de réalisation, la composition stable selon l'invention est caractérisée en ce qu'elle comprend en outre au moins une immunoglobine.

Dans un mode de réalisation, la composition stable selon l'invention est caractérisée en ce qu'elle comprend en outre au moins un facteur de croissance.

Dans un mode de réalisation, la composition stable selon l'invention est caractérisée en ce qu'elle comprend en outre au moins un composé choisi dans le groupe constitué de la lactoferrine, du lysozyme, des immunoglobines, ainsi qu'au moins un facteur de croissance.

Dans un mode de réalisation, la composition stable selon l'invention est caractérisée en ce qu'elle comprend en outre au moins un composé choisi dans le groupe constitué des huiles, des agents d'étalement, des émulsifiant, des lubrifiants, des adhésifs et leurs mélanges.

Dans un mode de réalisation, la composition stable selon l'invention est caractérisée en ce qu'elle comprend en outre au moins un composé choisi dans le groupe constitué du lauryl sulfate de sodium, du stéarate de magnésium, de la lécithine, des alcools éthoxylés, des huiles végétales, des huiles minérales, des huiles animales, des hexaoléate de polyoxyéthylène sorbitol, de la carboxyméthylcellulose (CMC), des gommes de xanthane, des gomme arabiques, de l'amidon et de leurs mélanges.

Dans un mode de réalisation, la composition stable selon l'invention est caractérisée en ce qu'elle comprend en outre au moins un composé choisi dans le groupe constitué du lauryl sulfate de sodium, du stéarate de magnésium, de la lécithine, des alcools éthoxylés, des huiles végétales, des huiles végétales phosphatidyle, des huiles minérales, des huiles animales, des hexaoléate de polyoxyéthylène sorbitol, de la carboxyméthylcellulose (CMC), des gommes de xanthane, des gommes arabiques, de l'amidon et de leurs mélanges, ce qui lui permet de renforcer par exemple la stabilité de l'adhérence lors de certaines application.

L'invention concerne également une composition stable comprenant des entités chimiques comprenant des atomes d'iode, dans laquelle les entités les plus présentes en nombre et qui incluent au moins un atomes d'iode sont choisies dans le groupe constitué des ions I₂SCN⁻, I(SCN)₂⁻ et leurs mélanges.

L'invention concerne également une composition stable comprenant des entités chimiques comprenant des atomes d'iode, dans laquelle au moins 50 % des atomes d'iode de ladite composition sont engagés dans des ions choisis dans le groupe constitué des ions I₂SCN⁻, I(SCN)₂⁻, et leurs mélanges.

L'invention concerne également un procédé de fabrication d'une composition selon l'invention, comprenant :
- une étape A de préparation d'un milieu réactionnel comprenant au moins deux substrats, au moins un oxydant, et un catalyseur, la mise en présence dudit catalyseur et dudit oxydant étant subordonnée à la mise en présence desdits deux substrats ;
- une étape B de réaction débutant à la mise en présence dudit oxydant et dudit catalyseur ;
- une étape C de retrait dudit catalyseur, et de récupération d'une composition selon l'invention comprenant au moins des ions I₂SCN⁻ et/ou des ions I(SCN)₂⁻ ;
   lesdits substrats étant des ions halogénure (X⁻) et thiocyanate (SCN⁻),
   ledit oxydant étant un système générateur de peroxyde d'hydrogène (H₂O₂) et/ou du peroxyde d'hydrogène, le catalyseur étant au moins une peroxydase,
   ledit procédé étant caractérisé en ce que ladite étape de réaction a une durée comprise entre 30 et 1800 secondes et en ce qu'il ne donne pas lieu à la formation d'ion hypothiocyanite (OSCN⁻).

Dans un mode de réalisation ladite mise en présence desdits substrats est simultanée.

Dans un mode de réalisation ladite mise en présence desdits substrats est séquentielle.

Selon la présente invention, les ions halogénures sont des ions iodures.

Dans un mode de réalisation, le procédé selon l'invention est caractérisé en ce que la composition selon l'invention récupérée à l'issue de l'étape C comprend des ions I₂SCN⁻ et/ou des ions I(SCN)₂⁻.

Dans un mode de réalisation, le procédé selon l'invention est caractérisé en ce que le milieu réactionnel est une solution aqueuse.

Dans un mode de réalisation, le procédé selon l'invention est caractérisé en ce que lesdits ions halogénure (X⁻) et thiocyanate (SCN⁻) sont ajoutés audit milieu réactionnel sous forme de poudre ou de solution.

Dans un mode de réalisation, le procédé selon l'invention est caractérisé en ce que lesdits ions halogénure (X⁻) et thiocyanate (SCN⁻) sont ajoutés audit milieu réactionnel sous forme de poudre.

Dans un mode de réalisation, le procédé selon l'invention est caractérisé en ce que lesdits ions halogénure (X⁻) et thiocyanate (SCN⁻) sont ajoutés audit milieu réactionnel sous forme de solution.

Dans un mode de réalisation, le procédé selon l'invention est caractérisé en ce que lesdits ions halogénure (X⁻) et thiocyanate (SCN⁻) sont ajoutés audit milieu réactionnel sous forme, respectivement, de poudre et de solution ou de solution et de poudre.

Dans un mode de réalisation, le procédé selon l'invention est caractérisé en ce que ledit milieu obtenu à l'issue de l'étape C est une composition selon l'invention qui est stable.

Dans un mode de réalisation particulier, le procédé de fabrication d'une composition antimicrobienne, comprend au moins les étapes suivantes :
- Etape 1 : préparation d'une solution A1 comprenant au moins un ion halogénure (X⁻) ;
- Etape 2 : préparation d'une solution A2 comprenant au moins un ion thiocyanate (SCN⁻) ;
- Etape 3 : préparation d'un composant B constitué d'un système générateur de peroxyde d'hydrogène (H₂O₂), ou de peroxyde d'hydrogène en solution aqueuse ;
- Etape 4 : trempage de la peroxydase dans une solution aqueuse ou tamponnée ;
- Etape 5 : mise au contact du composant A1 et A2 dans la solution qui contient la peroxidase ;
- Etape 6 : mise au contact du composant B dans la solution obtenue à l'étape 5 et maintien du contact pendant un temps compris entre 30 et 1800 secondes ;
- Etape 7 : retrait de la peroxydase, et récupération d'une composition selon
l'invention comprenant au moins des ions X₂SCN⁻ et/ou des ions X(SCN)₂⁻, caractérisé en ce que ledit procédé ne donne pas lieu à la formation d'ion hypothiocyanite (OSCN⁻).

Les étapes 1, 2, 3, 4 et 5 pouvant être réalisées dans n'importe quel ordre ou simultanément.

Dans un mode de réalisation, les ions halogénures sont des ions iodures.

Dans un mode de réalisation particulier, le procédé de fabrication d'une composition antimicrobienne, comprend au moins les étapes suivantes :
- Etape 1 : préparation d'une solution A1 comprenant au moins un ion iodure (I⁻) ;
- Etape 2 : préparation d'une solution A2 comprenant au moins un ion thiocyanate (SCN⁻) ;
- Etape 3 : préparation d'un composant B constitué d'un système générateur de peroxyde d'hydrogène (H₂O₂), ou de peroxyde d'hydrogène en solution aqueuse ;
- Etape 4 : trempage de la peroxydase dans une solution aqueuse ou tamponnée ;
- Etape 5 : mise au contact du composant A1 et A2 dans la solution qui contient la peroxidase ;
- Etape 6 : mise au contact du composant B dans la solution obtenue à l'étape 5 et maintien du contact pendant un temps compris entre 30 et 1800 secondes ;
- Etape 7 : retrait de la peroxydase, et récupération d'une composition selon
l'invention comprenant au moins des ions I₂SCN⁻ et/ou des ions I(SCN)₂⁻, caractérisé en ce que ledit procédé ne donne pas lieu à la formation d'ion hypothiocyanite (OSCN⁻).

Les étapes 1, 2, 3, 4 et 5 pouvant être réalisées dans n'importe quel ordre ou simultanément.

A l'issue du procédé la composition selon l'invention peut être soumise à une étape de lyophilisation à l'issue de laquelle on obtient un lyophilisat qui lors d'une remise en solution, permet de reconstituer une composition antimicrobienne selon l'invention qui comprend des ions X₂SCN⁻ et/ou des ions X(SCN)₂⁻, et est exempte d'ion hypothiocyanite (OSCN⁻).

A l'issue du procédé la composition selon l'invention peut être soumise à une étape de lyophilisation à l'issue de laquelle on obtient un lyophilisat qui lors d'une remise en solution, permet de reconstituer une composition antimicrobienne selon l'invention qui comprend des ions I₂SCN⁻ et/ou des ions I(SCN)₂⁻, et est exempte d'ion hypothiocyanite (OSCN⁻).

A l'issue du procédé la composition selon l'invention peut être soumise à une étape de lyophilisation à l'issue de laquelle un lyophilisat est obtenu, ledit lyophilisat permettant, lors d'une remise en solution, la reconstitution de ladite composition qui comprend en association des ions I₂SCN⁻ et I(SCN)₂⁻, et qui est exempte d'ion hypothiocyanite (OSCN⁻).

L'ion thiocyanate (SCN⁻) peut être apporté sous toute forme, par exemple sous forme de thiocyanate de potassium (KSCN) ou de sodium (NaSCN).

L'ion iodure (I⁻) peut être apporté sous toute forme, par exemple sous forme de iodure de potassium (KI) ou de sodium (NaI) ou encore sous forme de diiode (I₂). Dans un mode de réalisation, l'ion iodure (I⁻) est apporté sous forme de iodure de potassium (KI).

Dans un mode de réalisation, l'ion iodure (I⁻) est apporté sous forme de diiode (I₂).

Par « ne donne pas lieu à la formation d'ion hypothiocyanite (OSCN⁻) » ou « composition exempte d'ion hypothiocyanite (OSCN⁻) » on entend qu'aucun pic indiquant la formation d'ion hypothiocyanite (OSCN⁻) n'est observé en RMN et en chromatographie anionique lors de l'analyse de la composition.

La composition antimicrobienne obtenue est stable, on entend par « stable » une composition qui ne perd que très peu ses propriétés au cours du temps. Il peut par exemple s'agir d'une composition antimicrobienne perdant moins de 20% de son activité en 5 mois lorsqu'elle est stockée en récipient fermé, à 4°C, à l'abri de la lumière.

Selon la présente invention, le procédé de fabrication d'une composition stable selon l'invention est caractérisé en ce que l'ion halogénure (X⁻) est l'ion iodure (I).

Dans un mode de réalisation, le procédé de fabrication d'une composition stable selon l'invention est caractérisé en ce que l'ion iodure (I⁻) est sous forme d'iodure de potassium (KI) ou d'iodure de sodium (NaI) ou de diiode.

Dans un mode de réalisation, le procédé de fabrication d'une composition stable selon l'invention est caractérisé en ce que l'ion iodure (I⁻) est sous forme d'iodure de potassium (KI).

Dans un mode de réalisation, le procédé de fabrication d'une composition stable selon l'invention est caractérisé en ce que l'ion iodure (I⁻) est sous forme d'iodure de sodium (NaI).

Dans un mode de réalisation, le procédé de fabrication d'une composition stable selon l'invention est caractérisé en ce que l'ion iodure (I⁻) est sous forme de diiode.

Dans un mode de réalisation, le procédé de fabrication d'une composition stable selon l'invention est caractérisé en ce que l'ion halogénure (X⁻) est présent à une concentration molaire comprise entre 0,1 mM et 1 M.

Dans un mode de réalisation, le procédé de fabrication d'une composition stable selon l'invention est caractérisé en ce que l'ion halogénure (X⁻) est présent à une concentration molaire comprise entre 0,1 mM et 500 mM.

Dans un mode de réalisation, le procédé de fabrication d'une composition stable selon l'invention est caractérisé en ce que l'ion halogénure (X⁻) est présent à une concentration molaire comprise entre 0,1 mM et 100 mM.

Dans un mode de réalisation, le procédé de fabrication d'une composition stable selon l'invention est caractérisé en ce que l'ion halogénure (X⁻) est présent à une concentration molaire comprise entre 0,1 mM et 10 mM.

Dans un mode de réalisation, le procédé de fabrication d'une composition stable selon l'invention est caractérisé en ce que l'ion halogénure (X⁻) est l'ion iodure (I⁻) et est présent à une concentration molaire comprise entre 0,1 mM et 10 mM.

Dans un mode de réalisation, le procédé de fabrication d'une composition stable selon l'invention est caractérisé en ce que l'ion thiocyanate (SCN⁻) est présent à une concentration molaire comprise entre 0,1 mM et 1 M.

Dans un mode de réalisation, le procédé de fabrication d'une composition stable selon l'invention est caractérisé en ce que l'ion thiocyanate (SCN⁻) est présent à une concentration molaire comprise entre 0,1 mM et 500 mM.

Dans un mode de réalisation, le procédé de fabrication d'une composition stable selon l'invention est caractérisé en ce que l'ion thiocyanate (SCN⁻) est présent à une concentration molaire comprise entre 0,1 mM et 100 mM.

Dans un mode de réalisation, le procédé de fabrication d'une composition stable selon l'invention est caractérisé en ce que l'ion thiocyanate (SCN⁻) est présent à une concentration molaire comprise entre 0,1 mM et 10 mM.

Dans un mode de réalisation, le procédé de fabrication d'une composition stable selon l'invention est caractérisé en ce que l'ion halogénure (X⁻) est l'ion iodure (I⁻), la concentration en ions iodure (I⁻) est supérieure à celle dudit ion thiocyanate (SCN⁻), et le pH de la solution est compris entre 4 et 8.

Dans un mode de réalisation, le procédé de fabrication d'une composition stable selon l'invention est caractérisé en ce que le rapport entre la concentration molaire en ion ion iodure (I⁻) et la concentration molaire en thiocyanate (SCN⁻) est strictement supérieur à 1.

Dans un mode de réalisation, le procédé de fabrication d'une composition stable selon l'invention est caractérisé en ce que le rapport entre la concentration molaire en ion ion iodure (I⁻) et la concentration molaire en thiocyanate (SCN⁻) est compris entre 1,5 et 40.

Dans un mode de réalisation, le procédé de fabrication d'une composition stable selon l'invention est caractérisé en ce que le rapport entre la concentration molaire en ion ion iodure (I⁻) et la concentration molaire en thiocyanate (SCN⁻) est compris entre 1,5 et 20.

Dans un mode de réalisation, le procédé de fabrication d'une composition stable selon l'invention est caractérisé en ce que le rapport entre la concentration molaire en ion ion iodure (I⁻) et la concentration molaire en thiocyanate (SCN⁻) est compris entre 1,5 et 20.

Dans un mode de réalisation, le procédé de fabrication d'une composition stable selon l'invention est caractérisé en ce que le rapport entre la concentration molaire en ion ion iodure (I⁻) et la concentration molaire en thiocyanate (SCN⁻) est compris entre 4 et 5.

Dans un mode de réalisation, le procédé de fabrication d'une composition stable selon l'invention est caractérisé en ce que le pH de la solution est compris entre 4 et 8.

Dans un mode de réalisation, le procédé de fabrication d'une composition stable selon l'invention est caractérisé en ce que le pH de la solution est compris entre 4,4 et 7,5.

Dans un mode de réalisation, le procédé de fabrication d'une composition stable selon l'invention est caractérisé en ce que ledit milieu est tamponné par un tampon choisi dans le groupe constitué par le tampon citrate, le tampon phosphate ou le tampon acétate.

Dans un mode de réalisation, le procédé de fabrication d'une composition stable selon l'invention est caractérisé en ce que ledit ion thiocyanate (SCN-) est sous forme thiocyanate de potassium (KSCN) ou thiocyanate de sodium (NaSCN).

Dans un mode de réalisation, le procédé de fabrication d'une composition stable selon l'invention est caractérisé en ce que ledit composant B est constitué d'un système générateur de peroxyde d'hydrogène (H₂O₂).

Dans un mode de réalisation, le procédé de fabrication d'une composition stable selon l'invention est caractérisé en ce que ledit composant B est constitué d'un système générateur de peroxyde d'hydrogène (H₂O₂) étant un système glucose oxydase (GOD) / Glucose (Glc).

Dans un mode de réalisation, le procédé de fabrication d'une composition stable selon l'invention est caractérisé en ce que la concentration molaire en peroxyde d'hydrogène est sensiblement égale à la somme des concentrations en ion thiocyanate (SCN⁻) et en ion halogénure (X⁻).

Dans un mode de réalisation, le procédé de fabrication d'une composition stable selon l'invention est caractérisé en ce que la concentration molaire en peroxyde d'hydrogène est sensiblement égale à la somme des concentrations en ion thiocyanate (SCN⁻) et en ion halogénure (X⁻) étant l'iodure (I⁻).

Dans un mode de réalisation, le procédé de fabrication d'une composition stable selon l'invention est caractérisé en ce que, le temps de mise en contact de l'oxydant et du catalyseur en présence des substrats ou le temps de réaction (étape B ou étape 6) est compris entre 30 et 1800 secondes ;

Dans un mode de réalisation, le procédé de fabrication d'une composition stable selon l'invention est caractérisé en ce que, le temps de mise en contact de l'oxydant et du catalyseur en présence des substrats ou le temps de réaction (étape B ou étape 6) est compris entre 30 et 900 secondes.

Dans un mode de réalisation, le procédé de fabrication d'une composition stable selon l'invention est caractérisé en ce que, le temps de mise en contact de l'oxydant et du catalyseur en présence des substrats ou le temps de réaction (étape B ou étape 6) est compris entre 30 et 200 secondes.

Dans un mode de réalisation, le procédé de fabrication d'une composition stable selon l'invention est caractérisé en ce que, le temps de mise en contact de l'oxydant et du catalyseur en présence des substrats ou le temps de réaction (étape B ou étape 6) est compris entre 30 et 100 secondes.

Dans un mode de réalisation, le procédé de fabrication d'une composition stable selon l'invention est caractérisé en ce que, le temps de mise en contact de l'oxydant et du catalyseur en présence des substrats ou le temps de réaction (étape B ou étape 6) est compris entre 50 et 100 secondes.

Dans un mode de réalisation, le procédé de fabrication d'une composition stable selon l'invention est caractérisé en ce que ledit retrait de ladite peroxydase est réalisé au moyen d'une méthode choisie dans le groupe constitué de l'utilisation d'un « sachet de thé », de la centrifugation, de la floculation, de la mise en contact avec un support sur lequel est greffé la peroxydase comme par exemple des fibres, un textile, des résines polymériques, des granulés etc.

Dans un mode de réalisation, le procédé de fabrication d'une composition stable selon l'invention est caractérisé en ce que ledit retrait de ladite peroxydase est réalisé au moyen de l'utilisation d'un « sachet de thé ».

Dans un mode de réalisation, le procédé de fabrication d'une composition stable selon l'invention est caractérisé en ce que la peroxydase est choisie dans le groupe constitué par la lactoperoxydase (LP), la peroxydase thyroïdienne (TPO), la myéloperoxydase (MPO), la peroxydase salivaire (SPO) et la peroxydase éosinophile (EPO).

Dans un mode de réalisation, le procédé de fabrication d'une composition stable selon l'invention est caractérisé en ce que la peroxydase est la lactoperoxydase (LP).

La lactoperoxydase est une poudre que l'on peut mélanger par exemple avec de la bentonite ou fixer en solution liquide sur des billes de résines cationiques. Ces supports peuvent être placés dans un « sachet de thé ».

Pour la fixation de la LPO sur des billes de résine cationiques, certaines billes fixent +/- 40 mg de LPO par ml de résine.

Dans un mode de réalisation, le procédé de fabrication d'une composition stable selon l'invention est caractérisé en ce que ladite peroxydase a une concentration comprise 1 mg/L et 500 mg/L.

Dans un mode de réalisation, le procédé de fabrication d'une composition stable selon l'invention est caractérisé en ce que ladite peroxydase a une concentration comprise 50 mg/L et 250 mg/L.

Dans un mode de réalisation, le procédé de fabrication d'une composition stable selon l'invention est caractérisé en ce qu'il comprend en outre au moins une étape d'immobilisation sur un substrat immobilisant de la composition selon l'invention.

Un « substrat immobilisant », dans le contexte de la présente demande, est un matériau permettant de retenir ladite composition lors de la manipulation, cette dernière étant sous forme liquide, ou sous forme seche (résidu sec issu suite à l'évaporation de la composition selon l'invention, ou suite à lyophilisation).

Dans un mode de réalisation, le substrat immobilisant est un matériau fibreux.

Dans un mode de réalisation, le substrat immobilisant est un tissu.

Dans un mode de réalisation, le substrat immobilisant est un tissu imbibé.

Lors d'une remise en solution ou appliqué sur des micro-organismes, le substrat immobilisant permet de reconstituer une composition antimicrobienne selon l'invention.

L'invention concerne également un substrat immobilisant comprenant une composition selon l'invention.

L'invention concerne également des utilisations dans des buts prophylactiques et/ou thérapeutiques de la composition stable selon l'invention.

Dans un mode de réalisation, la composition stable selon l'invention est caractérisée en ce qu'elle est utilisée dans le traitement des infections.

Dans un mode de réalisation, la composition stable selon l'invention est caractérisée en ce qu'elle est utilisée en tant qu'antibactérien.

Dans un mode de réalisation, la composition stable selon l'invention est caractérisée en ce qu'elle est utilisée en tant qu'antiviral.

Dans un mode de réalisation, la composition stable selon l'invention est caractérisée en ce qu'elle est utilisée en tant qu'antifongique.

Dans un mode de réalisation, la composition stable selon l'invention est caractérisée en ce qu'elle est utilisée pour détruire un micro-organisme choisi dans le groupe constitué de *Colletotrichum lindemuthanium, Fusarium avenaceum, Septoria tritici, Verticillium dahliae, Phytophthora infestans, Pythium ultimum, Colletotrichum musae, Pencillium italicum, Pénicillium digitatum, Botrytis cinerea, Pénicillium expansum, Pectobacterium atroseptica, Pseudomonas syringae pv syringae, Pectobacterium carotovorum, Erwynia amylovora, Pseudomonas syringae pv. tomato, Clavibacter michiganensis subsp. Michiganensis, Kocuria rhizolia, Staphylococcus aureus, Enterobacter gergoviae, Escherichia coli, Klesiella pneumoniae, Pseudomonas aeruginosa, Pseudomonas fluorescent, Pseudomonas putita, Aspergillus niger, Penicillium pinophilum, Candida albicans Burhholderia cepacia, Pseudomonas aeruginosa, Staphylococcus aureus, Klebsiella oxytoca, Burkholderia multivorans, Achromobacter denitrificans, Pseudomonas aeruginosa, Srenotrophomonas maltophilia, Rhodococcus equi, Streptococcus equi, Streptococcus mutans* et *Streptococcus zooépidemicus*

Dans un mode de réalisation, la composition stable selon l'invention est caractérisée en ce qu'elle est utilisée pour détruire un micro-organisme choisi dans le groupe constitué de *Colletotrichum lindemuthanium, Fusarium avenaceum, Septoria tritici, Verticillium dahliae, Phytophthora infestans, Pythium ultimum, Colletotrichum musae, Pencillium italicum, Pénicillium digitatum, Botrytis cinerea, Penicillium expansum, Pectobacterium carotovorum, Pseudomonas syringae pv syringae, Pectobacterium atroseptica, Erwynia amylovora, Pseudomonas syringae pv. tomato, Clavibacter michiganensis subsp. Michiganensis, Kocuria rhizolia, Staphylococcus aureus, Enterobacter gergoviae, Escherichia coli, Klesiella pneumoniae Pseudomonas aeruginosa, Pseudomonas fluorescent, Pseudomonas putita, Aspergillus niger, Penicillium pinophilum,* et *Candida albicans .*

Dans un mode de réalisation, la composition stable selon l'invention est caractérisée en ce qu'elle est utilisée pour détruire *Xylella fastidiosa.*

Dans un mode de réalisation, la composition stable selon l'invention est caractérisée en ce qu'elle est utilisée pour détruire un micro-organisme choisi dans le groupe constitué de *Veillonella Alcalescens, Fusobacterium Nucleatum, Actinomyces Viscosus, Lactobacillus Acidophilus, Streptococcus Mutans, Porphyromonas gingivalis, Prevotella intermedia, Campylobacter species, Treponema socranskii species, Streptococcus species, Eikenella, Capnocytophaga species,* et *Selenomonas species.*

Dans un mode de réalisation, la composition stable selon l'invention est caractérisée en ce qu'elle est utilisée pour détruire un micro-organisme choisi dans le groupe constitué de *Veillonella Alcalescens, Fusobacterium Nucleatum, Actinomyces Viscosus, Lactobacillus Acidophilus* et *Streptococcus Mutans.*

Dans un mode de réalisation, la composition stable selon l'invention est caractérisée en ce qu'elle est utilisée pour détruire au moins un micro-organisme choisi dans le groupe des bactéries organisées en biofilm.

Dans un mode de réalisation, la composition stable selon l'invention est caractérisée en ce qu'elle est utilisée pour détruire au moins un micro-organisme choisi dans le groupe des bactéries organisées en biofilm, responsables des parodontites, choisi dans le groupe constitué de *Veillonella Alcalescens, Fusobacterium Nucleatum, Actinomyces Viscosus, Lactobacillus Acidophilus* et de *Streptococcus Mutans.*

Dans un mode de réalisation, la composition stable selon l'invention est caractérisée en ce qu'elle est utilisée pour détruire au moins un micro-organisme comme le Candida albicans, organisé en biofilm ou sous forme isolée, responsable de la glossite, du muguet, de la stomatite du dentier, de la chéilite, de la perlèche, des mycoses des pieds et des ongles.

Dans un mode de réalisation, la composition stable selon l'invention est caractérisée en ce qu'elle n'a pas d'effet sur *streptococcus salivarius.*

Dans un mode de réalisation, la composition stable selon l'invention est caractérisée en ce qu'elle est utilisée en tant que médicament chez l'Homme et/ou l'animal.

Dans un mode de réalisation, la composition stable selon l'invention est caractérisée en ce qu'elle est utilisée en tant que médicament chez l'Homme.

Dans un mode de réalisation, la composition stable selon l'invention est caractérisée en ce qu'elle est utilisée en tant que médicament chez l'animal.

Dans un mode de réalisation, la composition stable selon l'invention est caractérisée en ce qu'elle est utilisée dans le traitement et/ou la prévention des infections chez l'Homme et/ou l'animal.

Dans un mode de réalisation, la composition stable selon l'invention est caractérisée en ce qu'elle est utilisée dans le traitement des infections chez l'Homme.

Dans un mode de réalisation, la composition stable selon l'invention est caractérisée en ce qu'elle est utilisée dans le traitement des infections chez l'animal.

Dans un mode de réalisation, la composition stable selon l'invention est caractérisée en ce qu'elle est utilisée dans la prévention des infections chez l'Homme.

Dans un mode de réalisation, la composition stable selon l'invention est caractérisée en ce qu'elle est utilisée dans la prévention des infections chez l'animal.

Dans un mode de réalisation, la composition stable selon l'invention est caractérisée en ce qu'elle est utilisée dans le traitement des infections chez l'Homme et/ou chez l'animal causées par au moins un micro-organisme.

Dans un mode de réalisation, la composition stable selon l'invention est caractérisée en ce qu'elle est utilisée dans le traitement des infections chez le cheval causées par au moins un micro-organisme.

Dans un mode de réalisation, la composition stable selon l'invention est caractérisée en ce qu'elle est utilisée dans le traitement et/ou la prévention des infections causée par un micro-organisme formant un biofilm et/ou sous forme dite « planctonique ».

Dans un mode de réalisation, la composition stable selon l'invention est caractérisée en ce qu'elle est utilisée dans le traitement et/ou la prévention des infections causée par un micro-organisme formant un biofilm sur la surface de cellules humaines choisies dans le groupe constitué des cellules de la peau, des cellules de la muqueuse orale, des cellules de la sphère orthorhinopharingique, des cellules de la sphère gastro-entérologique et des cellules de la sphère uro-génitale.

Dans un mode de réalisation, la composition stable selon l'invention est caractérisée en ce qu'elle est utilisée dans le traitement et/ou la prévention des infections causées par un micro-organisme choisi dans le groupe constitué des bactéries, des virus, des protozoaires, des levures, des moisissures, des champignons, des parasites et assimilés.

Dans un mode de réalisation, la composition stable selon l'invention est caractérisée en ce qu'elle est utilisée dans le traitement et/ou la prévention des infections causée par un micro-organisme qui est une bactérie.

Dans un mode de réalisation, la composition stable selon l'invention est caractérisée en ce qu'elle est utilisée dans le traitement et/ou la prévention des infections causée par un micro-organisme qui est une bactérie choisie dans le groupe constitué de *Shigella, Salmonella, E. coli, Vibreo colera, Pseudomonas (Ps. pyocyanea), Staphylococcus (Staph. albus, aureus), Streptococcus (Strep. viridans, Strep. faecalis, B Streptococcus), Proteus, Helicobacter pylori* et assimilés, préférablement *H. pylori.*

Dans un mode de réalisation, la composition stable selon l'invention est caractérisée en ce qu'elle est utilisée dans le traitement et/ou la prévention des infections causée par un micro-organisme qui est un virus.

Dans un mode de réalisation, la composition stable selon l'invention est caractérisée en ce qu'elle est utilisée dans le traitement et/ou la prévention des infections causée par un micro-organisme qui est un virus enveloppé.

Dans un mode de réalisation, la composition stable selon l'invention est caractérisée en ce qu'elle est utilisée dans le traitement et/ou la prévention des infections causée par un micro-organisme qui est un virus enveloppé choisi dans le groupe constitué des virus causant l'herpès (préférablement les paramyxovirus de l'herpès simplex comme par exemple les virus parainfluenza) des orthomyxovirus (comme par exemple le virus de la grippe A et B), des rotavirus, des coronavirus, des virus de l'herpès (comme par exemple le virus VZV, le cytomégalovirus, le virus Epstein-Barr et le virus HHV6) et des rétrovirus (comme par exemple le virus de leucémie à lymphocytes T humain 1, le virus de la leucémie bovine et le virus de l'immunodéficience simienne (VIS)).

Dans un mode de réalisation, la composition stable selon l'invention est caractérisée en ce qu'elle est utilisée dans le traitement de la plaque dentaire, des maladies périodontales, des ulcères à *Helicobacter pylori,* des infections connues sous le nom de « tourista », des vaginites bactériennes, des vaginoses, des cystites, des infections à chlamydia, des infections gastro-intestinales, de la diarrhée, des caries, des gingivites, des mucosités, de l'herpès, de l'acné et du molluscum contagiosum.

Dans un mode de réalisation, la composition stable selon l'invention est caractérisée en ce qu'elle est utilisée dans le traitement de la plaque dentaire, des maladies périodontales, des ulcères, des infections connues sous le nom de « tourista », des vaginites bactériennes, des vaginoses, des cystites et des infections à chlamydia.

Dans un mode de réalisation, la composition stable selon l'invention est caractérisée en ce qu'elle est utilisée dans le traitement de la plaque dentaire et en ce que ledit micro-organisme présent dans la sphère bucco-dentaire est *Candida albicans.*

Dans un mode de réalisation, la composition stable selon l'invention est caractérisée en ce qu'elle est utilisée dans le traitement de la mucoviscidose et que le micro-organisme présent est choisi dans le groupe constitué de *Burhholderia cepacia, Pseudomonas aeruginosa* et *Staphylococcus aureus.*

Dans un mode de réalisation, la composition stable selon l'invention est caractérisée en ce en ce qu'elle est administrée par voie orale, topique ou injectable.

Dans un mode de réalisation, la composition stable selon l'invention est caractérisée en ce en ce qu'elle est administrée par voie orale.

Dans un mode de réalisation, la composition stable selon l'invention est caractérisée en ce en ce qu'elle est administrée par voie topique.

Dans un mode de réalisation, la composition stable selon l'invention est caractérisée en ce en ce qu'elle est administrée par voie injectable.

Dans un mode de réalisation, la composition stable selon l'invention est caractérisée en ce en ce qu'elle est administrée sous forme d'un gel, d'un produit de rinçage de la bouche, d'une pâte dentifrice, de comprimés, de capsules de gélatine molle, de pastilles, de poudre, de mélanges de poudres, d'un tissu imbibé, etc.

Les compositions selon l'invention, peuvent comprendre, en plus des composés mentionnés ci-dessus, tout excipient pharmaceutiquement acceptable connu de l'homme de l'art. De tels matériaux devraient être non toxiques. La nature précise des excipients peut dépendre d'un certain nombre de facteurs, dont la voie d'administration.

Dans un contexte thérapeutique, i.e. lorsqu'un effet thérapeutique est recherché, la dose administrée correspond à la "dose thérapeutique", qui dépend de plusieurs facteurs (voie d'administration, âge du patient, sexe, etc.) connus de l'homme de l'art, ce dernier pouvant déterminer ladite dose.

Dans un mode de réalisation, la composition stable selon l'invention est caractérisée en ce qu'elle est utilisée dans le domaine de l'agriculture, de l'horticulture, de la culture de plantes destinées à être consommées, de la culture de plantes destinées à être exposées comme plantes ornementales, de la culture de plantes fruitières, de la culture à partir de bulbe, de la culture de plante en pot, de l'entretien de forêts, du traitement des fruits ou graines cueillis, du traitement des racines isolées, etc.

Dans un mode de réalisation, la composition stable selon l'invention est caractérisée en ce qu'elle est utilisée pour détruire un micro-organisme dans le traitement des pathologies de plantes causées par au moins un micro-organisme phytopathogène.

Dans un mode de réalisation, la composition stable selon l'invention est caractérisée en ce qu'elle est utilisée pour détruire un micro-organisme phytopathogène choisi dans le groupe constitué des bactéries, des virus et des champignons et assimilés.

Dans un mode de réalisation, la composition stable selon l'invention est caractérisée en ce qu'elle est utilisée pour le traitement de plantes contaminées par au moins un micro-organisme phytopathogène avant ou après la récolte.

Dans un mode de réalisation, la composition stable selon l'invention est caractérisée en ce qu'elle est utilisée pour le traitement de plantes contaminées par au moins un micro-organisme phytopathogène qui est une bactérie ou un virus.

Dans un mode de réalisation, la composition stable selon l'invention est caractérisée en ce qu'elle est utilisée pour le traitement de plantes contaminées par au moins un micro-organisme phytopathogène qui est une bactérie.

Dans un mode de réalisation, la composition stable selon l'invention est caractérisée en ce qu'elle est utilisée pour le traitement de plantes contaminées par au moins un micro-organisme phytopathogène qui est une bactérie choisie dans le groupe constitué de *Erwinia chrvsanthemi, Pseudomonas syringe, Xanthomonas camtestrise* et *Curtobactrium flaccumfaciens.*

Dans un mode de réalisation, la composition stable selon l'invention est caractérisée en ce qu'elle est utilisée pour le traitement de plantes contaminées par au moins un micro-organisme phytopathogène qui est choisi dans le groupe constitué de *Erwinia amylovora, Pectobacterium carotovorum subsp. Carotovorum, Pectobacterium atrosepticum, Pseudomonas synringae pv. syringae, Pseudomonas synringae pv. tomato* et *Clavibacter michiganensis subsp. Michiganensis.*

Dans un mode de réalisation, la composition stable selon l'invention est caractérisée en ce qu'elle est utilisée pour le traitement de plantes contaminées par au moins un micro-organisme phytopathogène qui est un champignon.

Dans un mode de réalisation, la composition stable selon l'invention est caractérisée en ce qu'elle est utilisée pour le traitement de plantes contaminées par au moins un micro-organisme phytopathogène choisi dans le groupe constitué de *Penicillium spp., Botryotinia spp.* comme par exemple *Botrytis cinerea, Didymella spp.* comme par exemple *Didymella lycopersici* ou *Didymella bryonia, Pythium spp., Plasmopara spp., Peronospora spp., Sclerospora spp., Sphaerotheca spp.* comme par exemple *Sphaerotheca pannose* et *Sphaerotheca fulisinea, Puccunia spp.* comme par exemple *Puccunia horiana, Erysiphe spp., Oidium spp., Leveillula spp.* comme par exemple *Leveillula taurica, Fusarium spp., Phytophtora spp., Rhizoctonia spp., Verticillium spp., Scierotinia spp., Rhizopus spp.* et *Ventura spp.*

Dans un mode de réalisation, la composition stable selon l'invention est caractérisée en ce qu'elle est utilisée pour le traitement de plantes contaminées par au moins un micro-organisme phytopathogène choisi dans le groupe constitué de *Colletotrichum lindemuthanium, Fusarium avenaceum, Septoria tritici, Verticillium dahlia, Phytophthora infestans, Pythium ultimum, Colletotrichum musae, Pénicillium italicum, Pénicillium digitatum, Botrytis cinerea et Penicillium expansum.*

Dans un mode de réalisation, la composition stable selon l'invention est caractérisée en ce qu'elle est utilisée pour le traitement de plantes contaminées par au moins un micro-organisme phytopathogène choisi dans le groupe constitué de *Colletotrichum lindemuthanium, Septoria tritici, Verticillium dahlia, Phytophthora infestans, Pythium ultimum, Colletotrichum musae, Penicillium italicum, Pénicillium digitatum, Botrytis cinerea et Pénicillium expansum.*

Dans un mode de réalisation, la composition stable selon l'invention est caractérisée en ce qu'elle est utilisée pour le traitement de plantes contaminées par au moins un micro-organisme phytopathogène qui est choisi dans le groupe constitué de *Botrytis cinerea, Penicillium expansum, Pénicillium italicum, Pénicillium digitatum, Fusarium avenaceum, Phytophthora infestans, Verticillium dahlia, Colletotrichum lindemuthanium, Colletotrichum musae, Pythium ultimum, Venturia inaequalis, Plasmopara viticola, Erysiphe necator, Pectobacterium caratovorum, Pectobacterium atrosepticum, Clavibacter michiganensis subsp. michiganensis, Pseudomonas syringae pv. tomato, Pseudomonas syringae subsp. syringae, Erwinia amylovora, Xanthomonas orizae,* et *Xylella fastidiosa.*

Dans un mode de réalisation, la composition stable selon l'invention est caractérisée en ce qu'elle est utilisée pour le traitement d'objet contaminés par au moins un micro-organisme pathogène choisi dans le groupe constitué de *Kocuria rhizolia, Staphylococcus aureus, Enterobacter gergoviae, Escherichia coli, Klesiella pneumoniae, Pseudomonas aeruginosa, Pseudomonas fluorescent, Pseudomonas putita, Aspergillus niger, Pénicillium pinophilum,* et *Candida albicans.*

Dans un mode de réalisation, la composition stable selon l'invention est caractérisée en ce qu'elle est utilisée en association avec un autre composé réputé combattre au moins un micro-organisme phytopathogène afin de pallier aux problèmes potentiels de résistance.

Dans un mode de réalisation, la composition stable selon l'invention est caractérisée en ce qu'elle est utilisée dans le traitement des plantes selon une méthode choisie dans le groupe constitué par la pulvérisation, l'arrosage, l'atomisation, la pulvérisation aérienne, l'arrosage, l'immersion, l'irrigation au goutte-à-goutte, le bain, etc.

Dans un mode de réalisation, la composition stable selon l'invention est caractérisée en ce qu'elle est utilisée pour la désinfection de systèmes d'irrigation au goutte-à-goutte.

Dans un mode de réalisation, la composition stable selon l'invention est caractérisée en ce qu'elle est sous une forme choisie dans le groupe constituée de la forme liquide et de la forme solide.

Dans un mode de réalisation, la composition stable selon l'invention est caractérisée en ce qu'elle est sous sous forme liquide.

Dans un mode de réalisation, la composition stable selon l'invention est caractérisée en ce qu'elle est sous forme solide.

### Description des figures

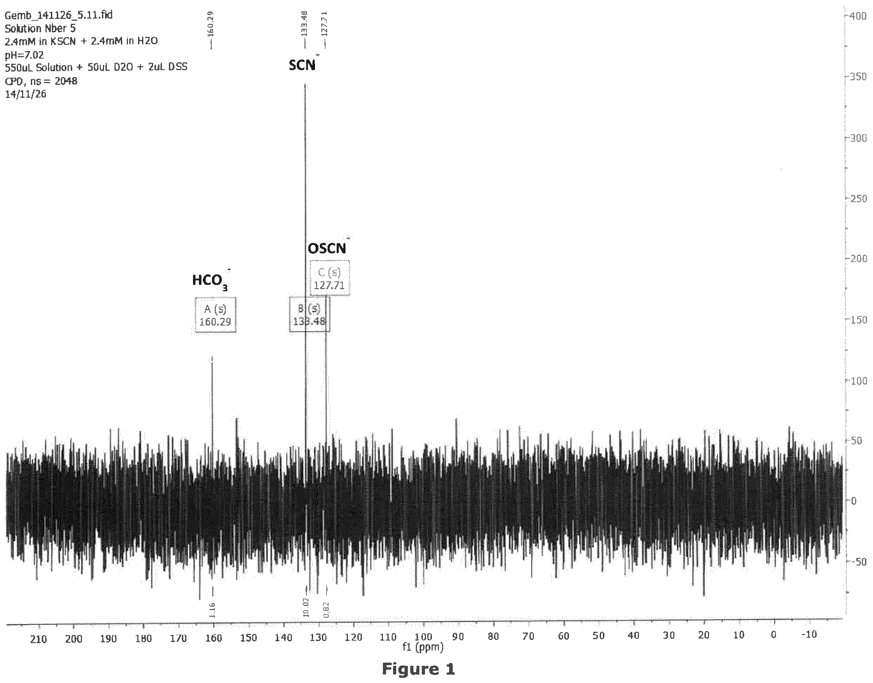

### Figure 1 : Spectre RMN d'une composition témoin sans iodure

La figure 1 est un spectre RMN ¹³C de la composition témoin sans iodure correspondant à l'essai 2 du tableau 1 (conditions : 2,4 mM KSCN + 2,4 mM H₂O₂ + LP ; tampon phosphate 100 mM pH 7,4). On observe sur ce spectre plusieurs pics très nets : à 133,48 ppm le pic correspond à l'ion thiocyanate (SCN⁻) et à 127,71 ppm le pic correspond à l'ion hypothiocyanite (OSCN⁻). Il n'y a aucun pic aux alentours de 50 ppm.

### Figure 2 : Spectre RMN d'une composition selon l'invention

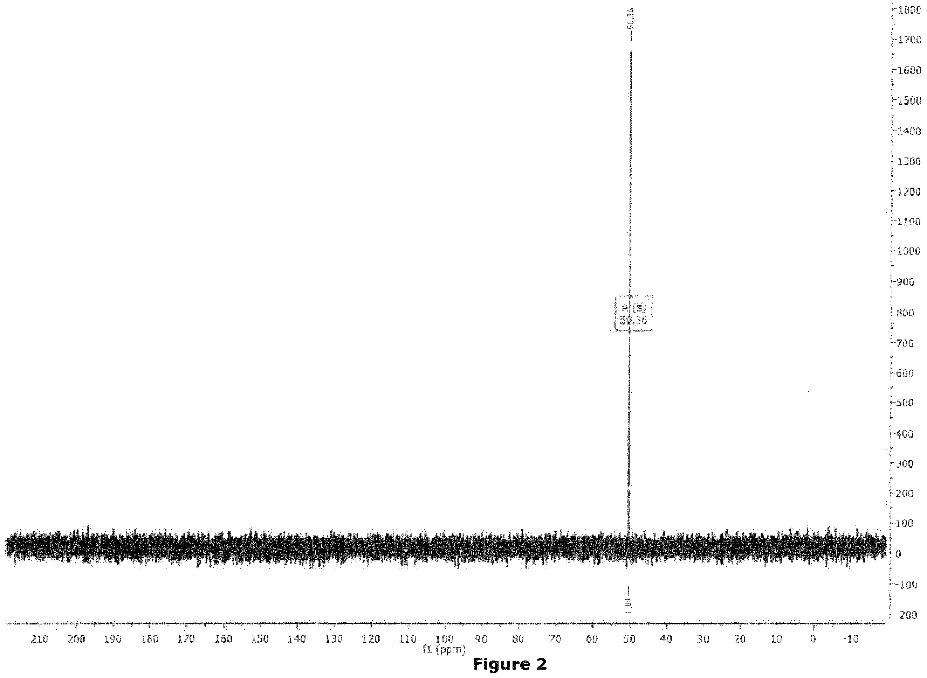

La figure 2 est un spectre RMN ¹³C de la composition correspondant à l'essai 20 du tableau 1 (conditions : 5,4 mM KI + 1,2 mM KSCN + 6,6 mM H₂O₂ + LP ; tampon phosphate 100 mM pH 7,4). On observe sur ce spectre le pic, positionné à 50,36 ppm pour cet essai, correspondant aux ions I₂SCN⁻ et I(SCN)₂⁻. Ce pic n'est pas observé dans le témoin sans KI (essai 2, voir figure 1). Aucun pic correspondant à l'ion thiocyanate (SCN⁻) ou à l'ion hypothiocyanite (OSCN⁻) n'est observable.

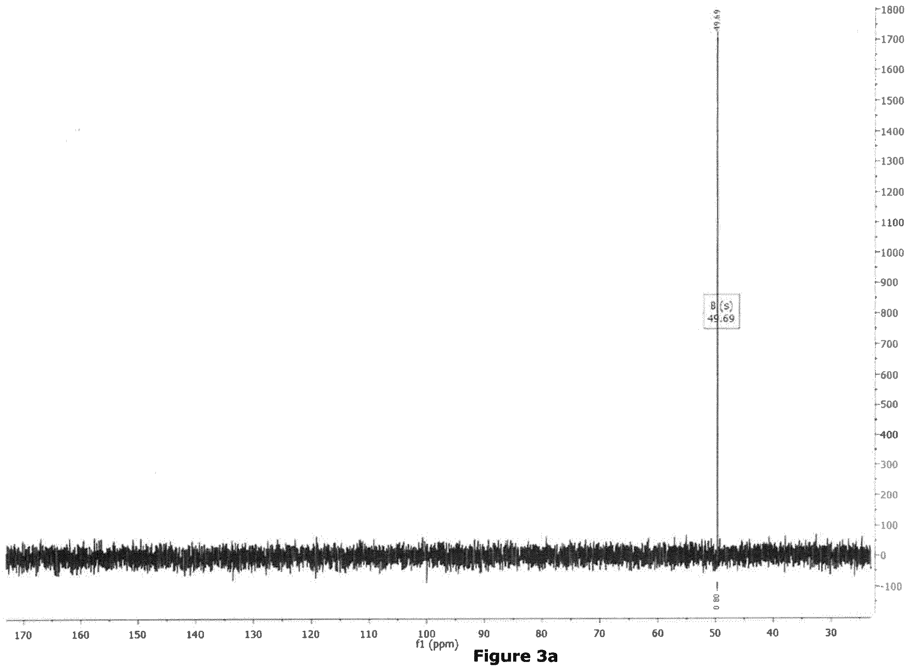

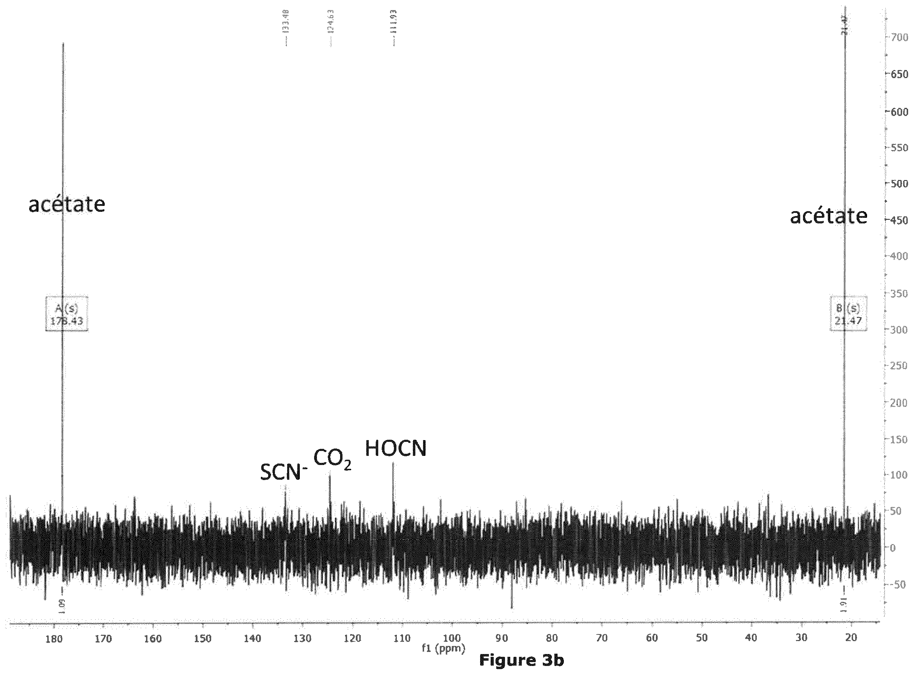

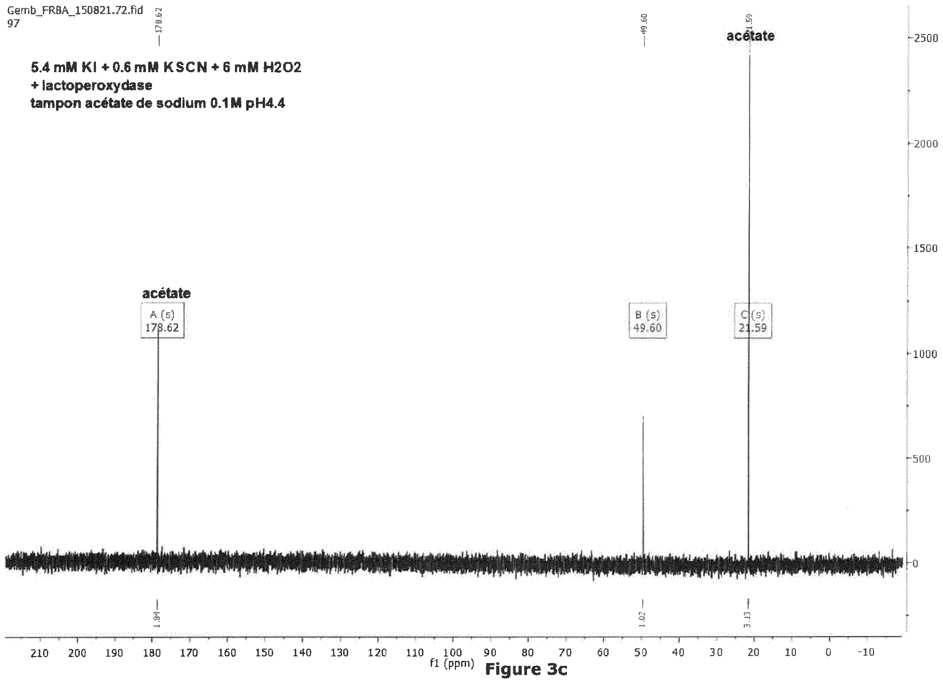

### Figures 3 : Spectre RMN d'une composition selon l'invention

**La** **figure 3a** est un spectre RMN ¹³C de la composition correspondant à l'essai 35 du tableau 2 (conditions : 5,4 mM KI + 1,2 mM KSCN + 6,6 mM H₂O₂ + LP ; tampon acétate de sodium 100 mM pH 4,5). Comme précisé ci-dessus, la concentration en H₂O₂ est égale à la somme de la concentration en KI et de la concentration en KSN. On observe sur ce spectre le pic, positionné à 49,6 ppm pour cet essai, correspondant aux ions I₂SCN⁻ et I(SCN)₂⁻. Aucun pic correspondant à l'ion thiocyanate (SCN⁻) ou à l'ion hypothiocyanite (OSCN⁻) n'est observable.

**La** **figure 3b** est un spectre RMN ¹³C de la composition correspondant à l'essai 33 du tableau 2 (conditions : 1,2 mM KI + 1,2 mM KSCN + 2,4 mM H₂O₂ + LP ; tampon acétate de sodium 100 mM pH 4,4). Comme précisé ci-dessus, la concentration en H₂O₂ est égale à la somme de la concentration en KI et de la concentration en KSN. On observe sur ce spectre aucun pic à 49,6 ppm pour cet essai, correspondant aux ions I₂SCN⁻ et I(SCN)₂⁻. Des pics correspondant à d'autres espèces sont en revanche observables.

**La** **figure 3c** est un spectre RMN ¹³C de la composition correspondant à l'essai 30 du tableau 2 (conditions : 5,4 mM KI + 0,6 mM KSCN + 6,0 mM H₂O₂ + LP ; tampon acétate de sodium 100 mM pH 4,4). Comme précisé ci-dessus, la concentration en H₂O₂ est égale à la somme de la concentration en KI et de la concentration en KSN. On observe sur ce spectre le pic, positionné à 49,6 ppm pour cet essai, correspondant aux ions I₂SCN⁻ et I(SCN)₂⁻. Aucun pic correspondant à l'ion thiocyanate (SCN⁻) ou à l'ion hypothiocyanite (OSCN⁻) n'est observable.

### Figure 4a : Spectre de masse d'une composition selon l'invention

La figure 4a est un spectre de masse d'une composition selon l'invention préparée avec du ¹²C (conditions : 5,4 mM KI + 5,4 mM H₂O₂ + LP ; tampon acétate d'ammonium 100 mM pH 4,5 - concentrations en KSCN testées 1,2 -2,4 - 4,8 - 5,4 - 7,2 - 10,6 - 0). Le fragment à 311,78 correspond à l'ion formé I₂S¹²CN⁻.

### Figure 4b : Spectre de masse d'une composition selon l'invention

La figure 4b est un spectre de masse d'une composition selon l'invention préparée avec du ¹³C. (conditions : 5,4 mM KI + 5,4 mM H₂O₂ + LP ; tampon acétate d'ammonium 100 mM pH 4,5 - concentrations en KSCN testées 1,2 -2,4 - 4,8 - 5,4 - 7,2 - 10,6). Le fragment à 312,78 correspond à l'ion formé I₂S¹³CN⁻.

### Figure 5 : Variation du taux de survie des candida albicans en fonction du temps

La figure 5 illustre la variation du taux de survie des *candida albicans* en fonction du temps, évaluée durant une période de 6 mois.

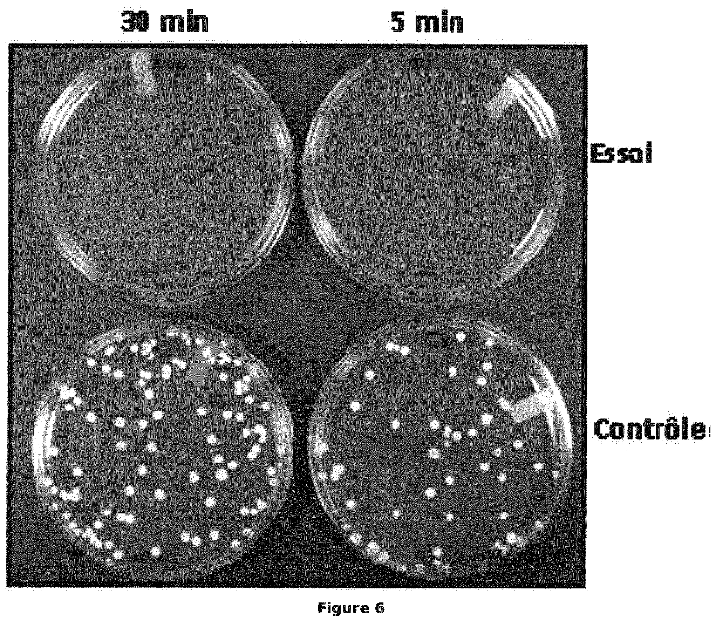

### Figure 6 : Photographies des milieux de culture après la mise en contact versus contrôle, après 5 minutes ou 30 minutes

La figure 6 consiste en des photographies des milieux de culture après la mise en contact versus contrôle. Que le temps de contact soit de 30 minutes (partie gauche de la figure) ou de 5 minutes (partie droite de la figure), le résultat visuel est le même : aucune colonie de candida albicans ne résiste à la composition selon l'invention.

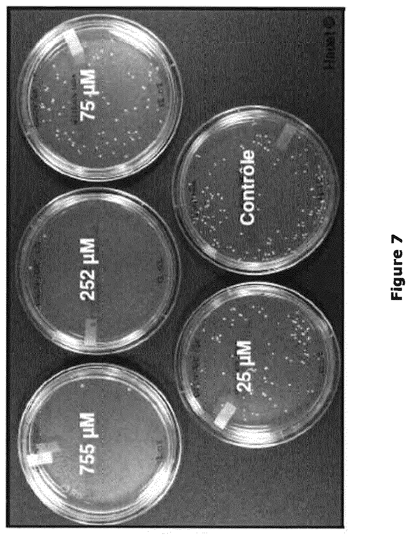

### Figure 7 : Photographies des milieux de culture après la mise en contact versus contrôle, à différentes concentrations

La figure 7 consiste en des photographies des milieux de culture après la mise en contact versus contrôle, à différentes concentrations. Il est observé que même à une concentration de 25 µm, le nombre de colonies formées est fortement diminué, par rapport au contrôle.

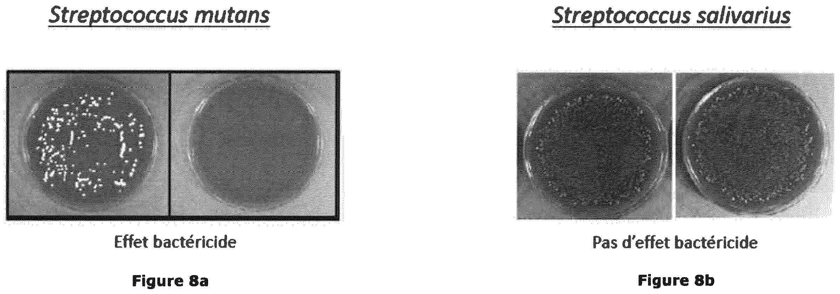

### Figure 8 : Sélectivité d'une composition selon l'invention

Les figures 8 sont des photographies des milieux de culture après la mise en contact avec *streptococcus mutans* (bactérie cariogène) Figure 8a et *streptococcus salivarius* (bactérie commensale) Figure 8b : il en est déductible que la composition selon l'invention a un effet bactéricide sur *streptococcus mutans* (Figure 8a) et n'a pas d'effet bactéricide sur *streptococcus salivarius* (Figure 8b).

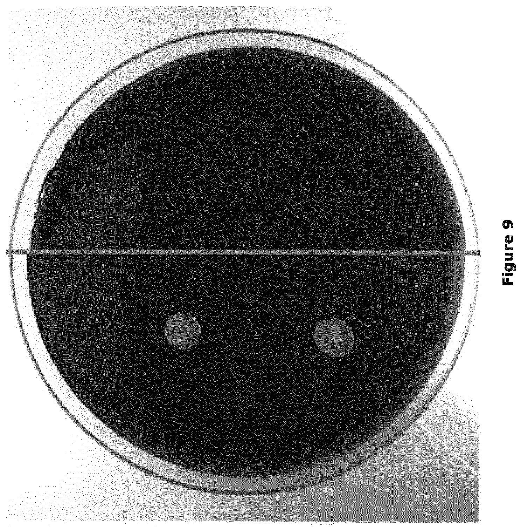

### Figure 9 : Activité sur Xylella fastidiosa subsp. fastidiosa

La figure 9 consiste en des photographies des milieux de culture après la mise en contact avec *Xylella fastidiosa subsp. fastidiosa:* il en est déductible que la composition selon l'invention a un effet bactéricide sur *Xylella fastidiosa subsp. fastidiosa* (partie droite de la figure) alors que le contrôle n'a pas d'effet (partie gauche de la figure).

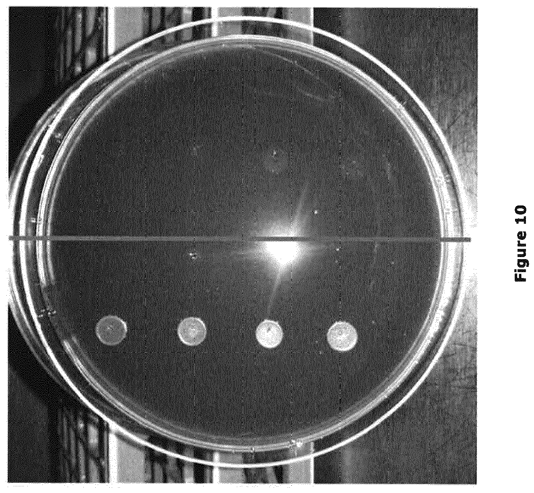

### Figure 10 : Activité sur Xylella fastidiosa subsp. multiplex

La figure 10 consiste en des photographies des milieux de culture après la mise en contact avec *Xylella fastidiosa subsp. multiplex:* il en est déductible que la composition selon l'invention a un effet bactéricide sur *Xylella fastidiosa subsp. multiplex* (partie droite de la figure) alors que le contrôle n'a pas d'effet (partie gauche de la figure).

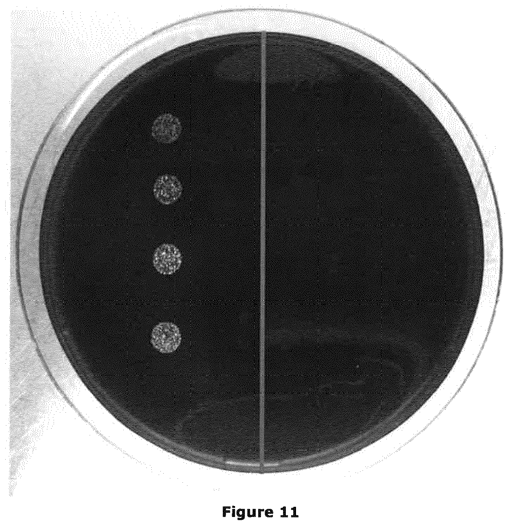

### Figure 11 : Activité sur Xylella fastidiosa subsp. pauca.

La figure 11 consiste en des photographies des milieux de culture après la mise en contact avec *Xylella fastidiosa subsp. pauca:* il en est déductible que la composition selon l'invention a un effet bactéricide sur *Xylella fastidiosa subsp. pauca* (partie droite de la figure) alors que le contrôle n'a pas d'effet (partie gauche de la figure).

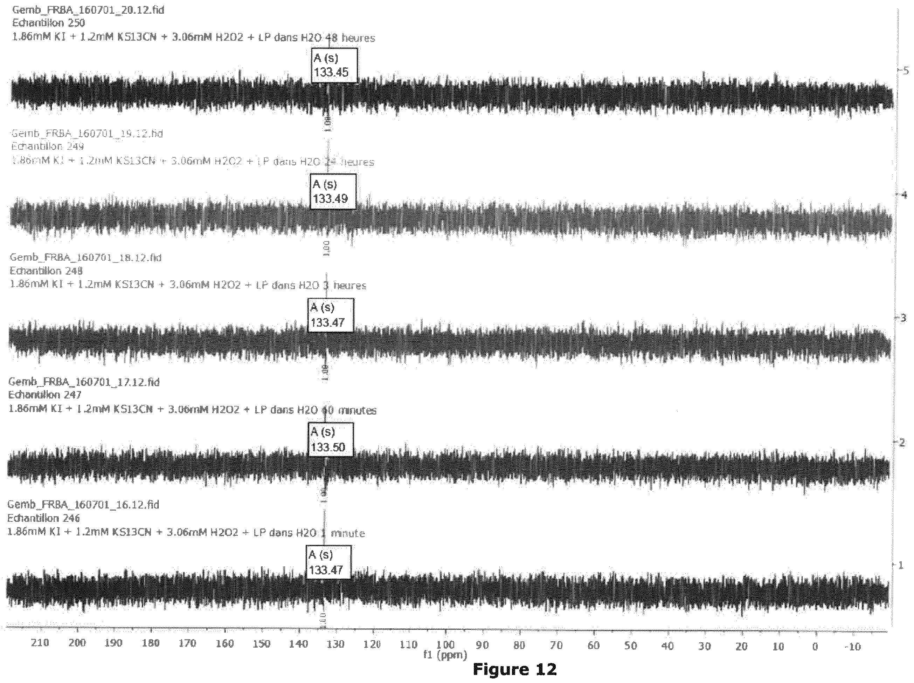

### Figure 12 : Absence des ions I₂SCN⁻ et/ou I(SCN)₂⁻ dans l'art antérieur (exemple 1 ; matrice aqueuse)

Des compositions obtenues par oxydation enzymatique d'un mélange halogènure thiocyanate ont été décrites dans l'art antérieur notamment dans EP1349457 ou WO2016026946. Des mélanges réalisés dans de l'eau selon les modes opératoires décrits dans ces demandes de brevet n'a permis d'obtenir un ion choisi dans le groupe constitué par les ions I₂SCN⁻ et/ou I(SCN)₂⁻.

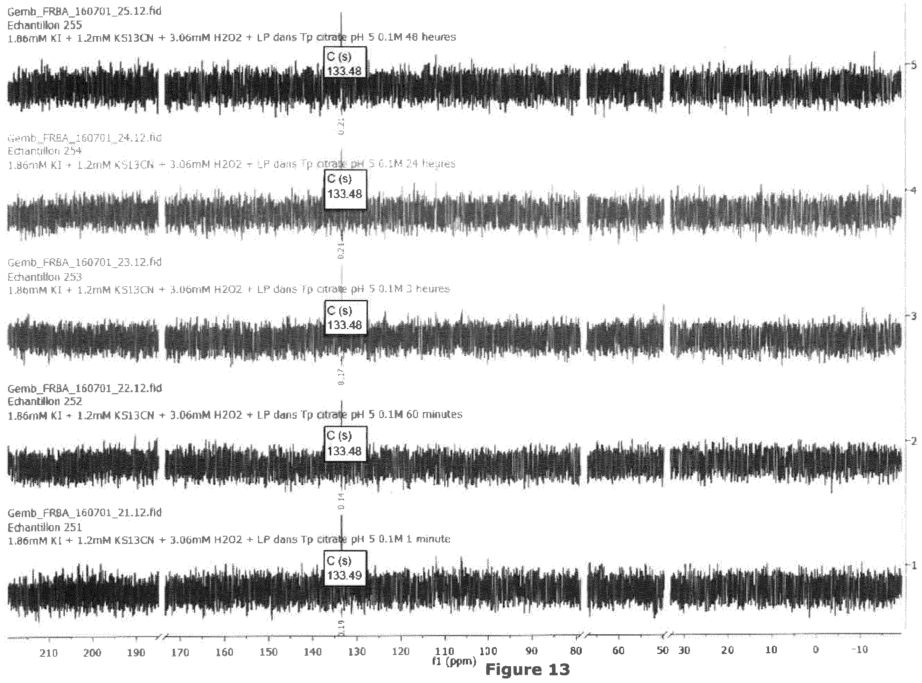

### Figure 13 : Absence des ions I₂SCN⁻ et/ou I(SCN)₂⁻ dans l'art antérieur (exemple 2 ; matrice tamponnée acide)

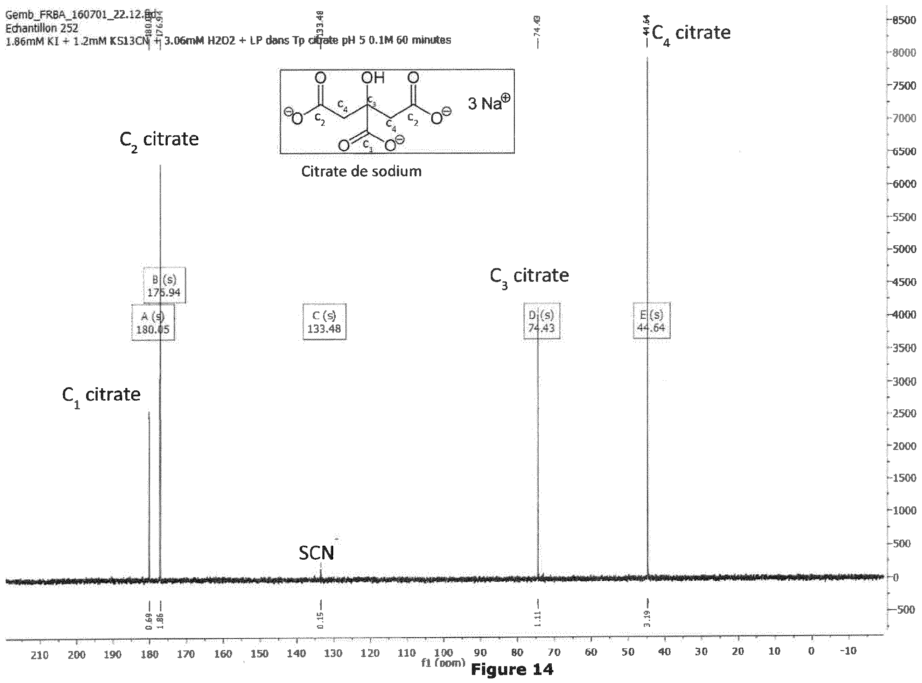

Des compositions obtenues par oxydation enzymatique d'un mélange halogènure thiocyanate ont été décrites dans l'art antérieur notamment dans EP1349457 ou WO2016026946. Des mélanges réalisés dans un tampon citrate 100 mM pH 5,5 selon les modes opératoires décrits dans ces demandes de brevet n'a permis d'obtenir un ion choisi dans le groupe constitué par les ions I₂SCN⁻ et/ou I(SCN)₂⁻. Pour plus de clarté les signaux correspondants au carbones du citrate de sodium ont été supprimés du spectre (voir figure 14).

### Figure 14 : Spectre RMN du citrate de sodium et du thiocyanate

Le citrate de sodium contient 4 carbones visibles en RMN aux déplacements chimiques suivants: C₁ sort à 180,45 ppm, C₂ sort à 175,94 ppm, C₃ sort à 73,43 ppm et C₄ sort à 44,64 ppm. Le thiocyanate contient 1 carbone, visible au déplacement chimique de 133,48 pppm.

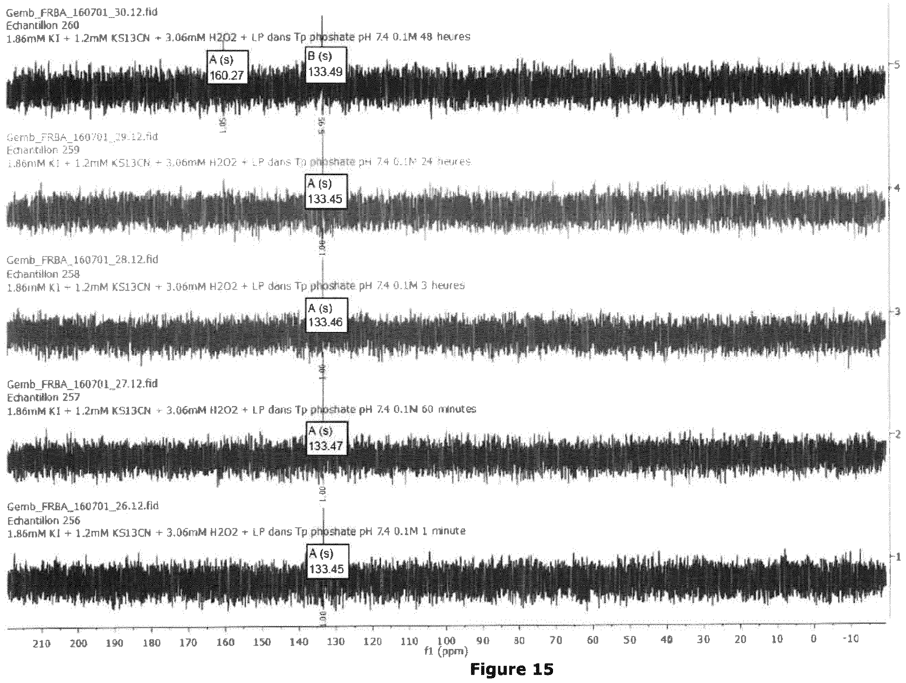

### Figure 15 : Absence des ions I₂SCN⁻ et/ou I(SCN)₂⁻ dans l'art antérieur (exemple 3 ; matrice tamponnée neutre)

Des compositions obtenues par oxydation enzymatique d'un mélange halogènure thiocyanate ont été décrites dans l'art antérieur notamment dans EP1349457 ou WO2016026946. Des mélanges réalisés dans un tampon phosphate 100 mM pH 7,4 selon les modes opératoires décrits dans ces demandes de brevet n'a permis d'obtenir un ion choisi dans le groupe constitué par les ions I₂SCN⁻ et/ou I(SCN)₂⁻.

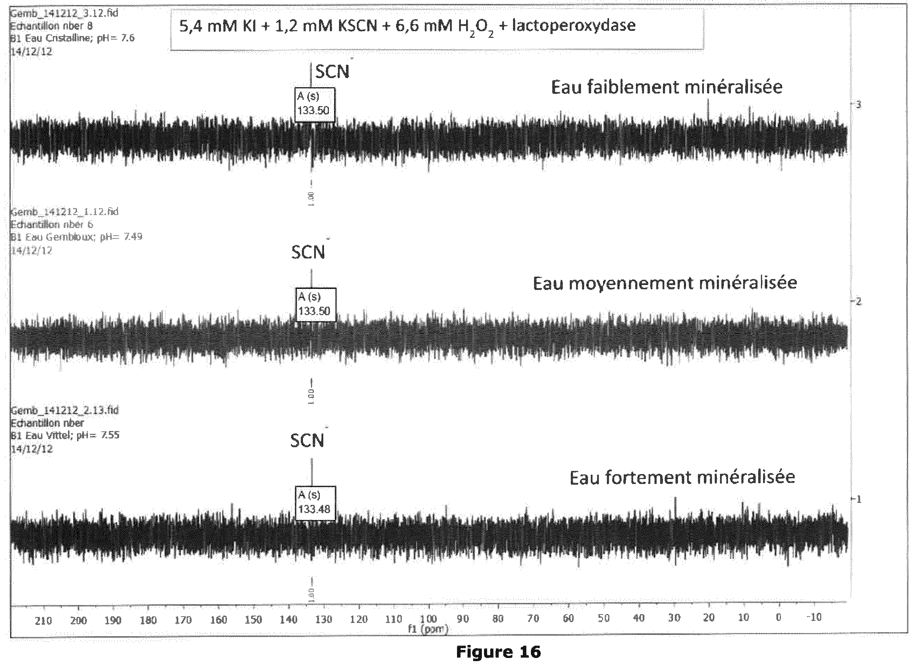

### Figure 16 : Importance de la matrice pour l'apparition des ions I₂SCN⁻ et/ou I(SCN)₂⁻

Des compositions obtenues par oxydation enzymatique d'un mélange halogènure thiocyanate dans le ratio idéal de 4,5 (KI par rapport à KSCN) dans une matrice aqueuse (type eau de source faiblement minéralisée, eau de source moyennement minéralisée, eau de source fortement minéralisée ou eau de ville), quelque soit la composition minérale de la matrice aqueuse, n'a pas permis d'obtenir un ion choisi dans le groupe constitué par les ions I₂SCN⁻ et/ou I(SCN)₂⁻.

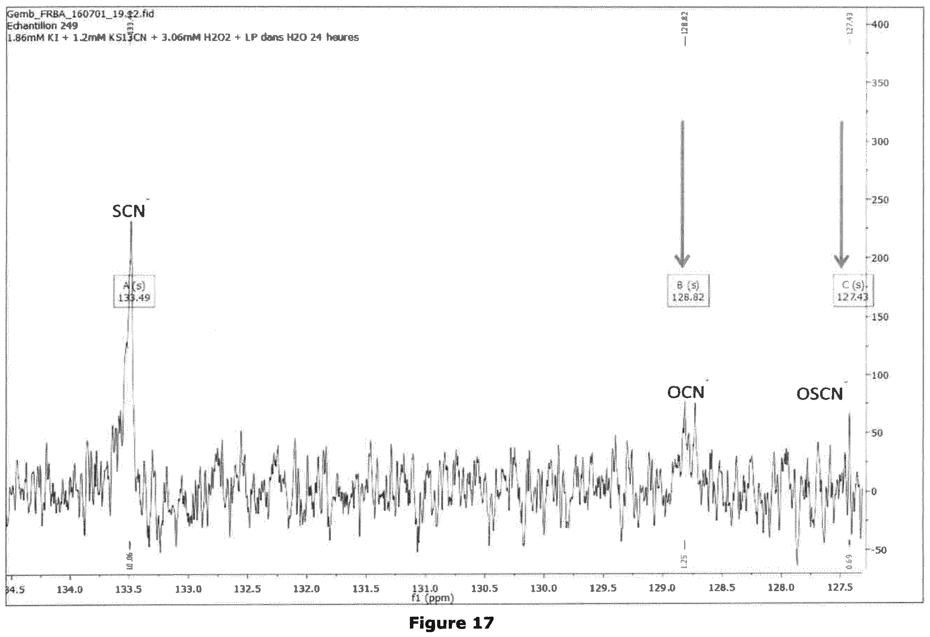

### Figure 17 : Présence des ions OSCN⁻ dans l'art antérieur (exemple 1 ; matrice aqueuse)

Des compositions obtenues par oxydation enzymatique d'un mélange halogènure thiocyanate ont été décrites dans l'art antérieur notamment dans EP1349457 ou WO2016026946. Des mélanges réalisés dans de l'eau selon les modes opératoires décrits dans ces demandes de brevet comprennent des ions hypothiocyanites.

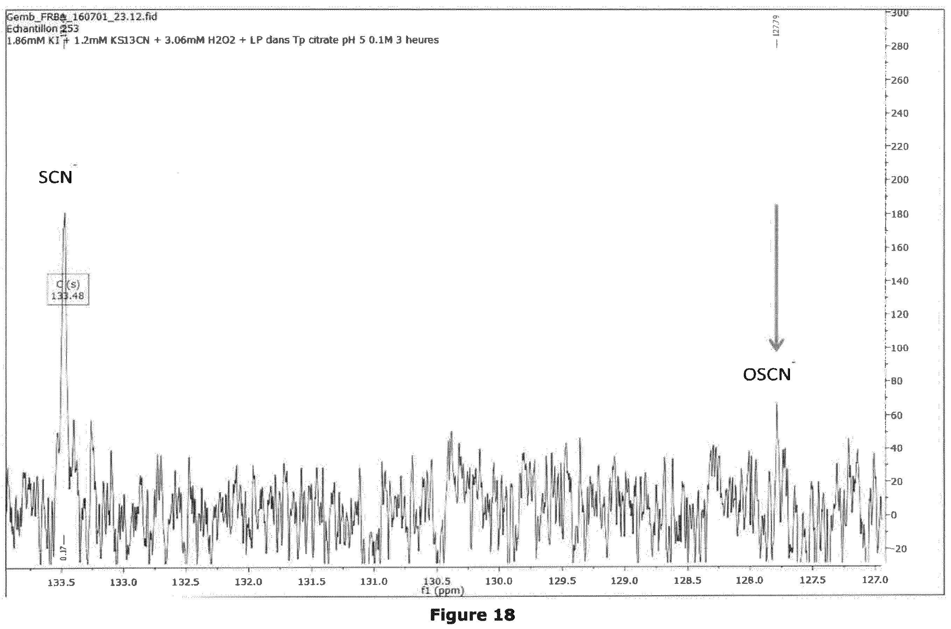

### Figure 18 : Présence des ions OSCN⁻ dans l'art antérieur (exemple 2 ; matrice tamponnée acide)

Des compositions obtenues par oxydation enzymatique d'un mélange halogènure thiocyanate ont été décrites dans l'art antérieur notamment dans EP1349457 ou WO2016026946. Des mélanges réalisés dans un tampon citrate 100 mM pH 5,5 selon les modes opératoires décrits dans ces demandes de brevet comprennent des ions hypothiocyanites.

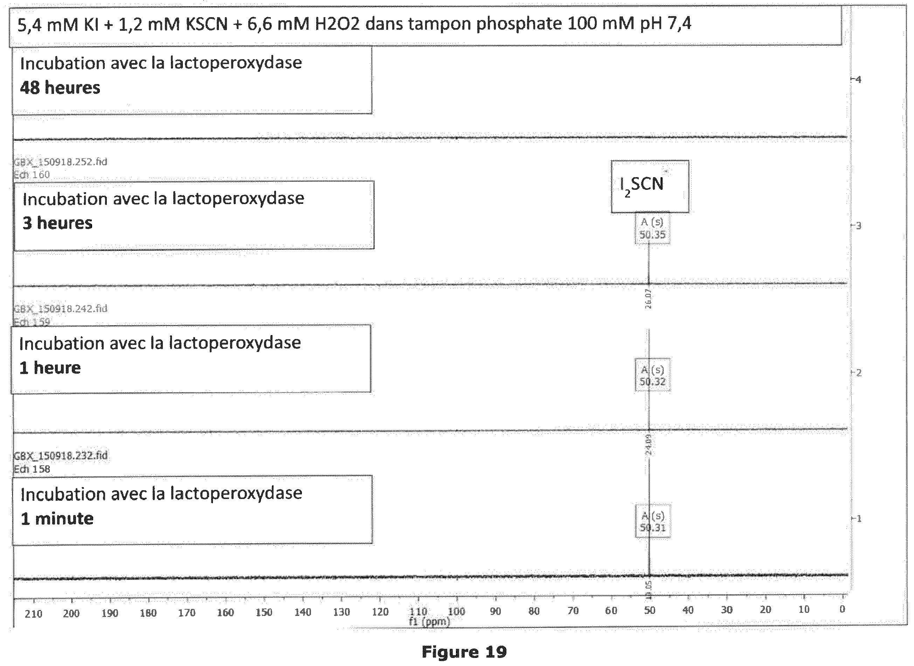

### Figure 19 : Oxydation enzymatique des ions I₂SCN⁻ et/ou I(SCN)₂⁻

La présence concommittante de la LP et des ions I₂SCN⁻ et/ou I(SCN)₂⁻ provoque une dégradation des ions I₂SCN⁻ et/ou I(SCN)₂⁻X avec une baisse du signal à 49 ppm après 1h (baisse légère), 3 h d'incubation (baisse prononcée du signal) et une disparition totale du signal après 48h.

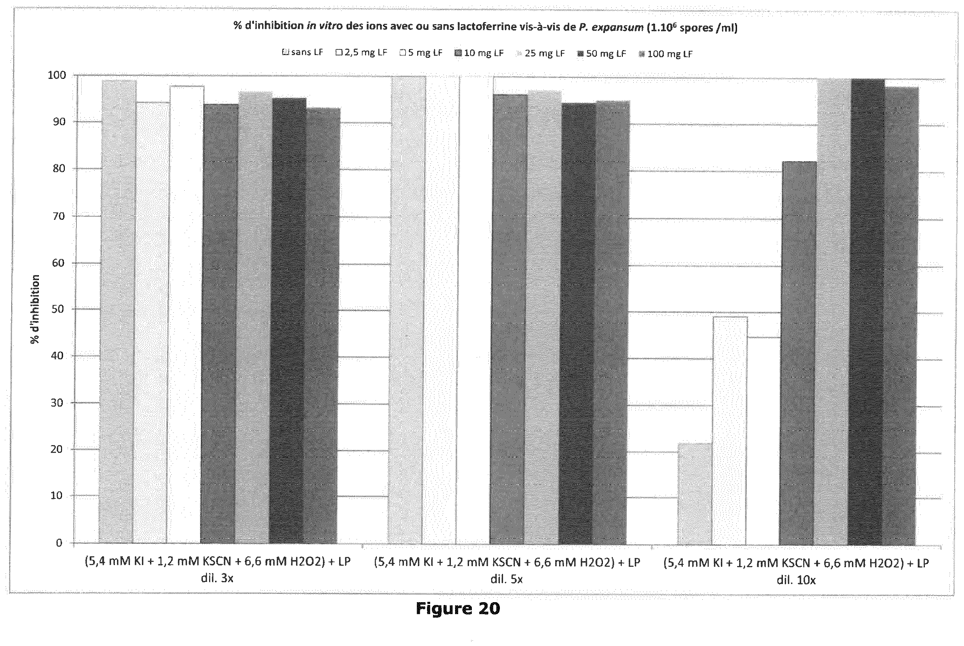

### Figure 20 : Effet de synergie entre la composition selon l'invention et la lactoferrine

La figure 20 illustre la synergie d'activité antimicrobienne vis-à-vis de P. *expansum* (10⁶ spores/ml) lorsque la composition selon l'invention est diluée 10 fois avec une ajoute de lactoferrine (> 2,5mgr/ml) comparée à la même solution sans ajoute de lactoferrine

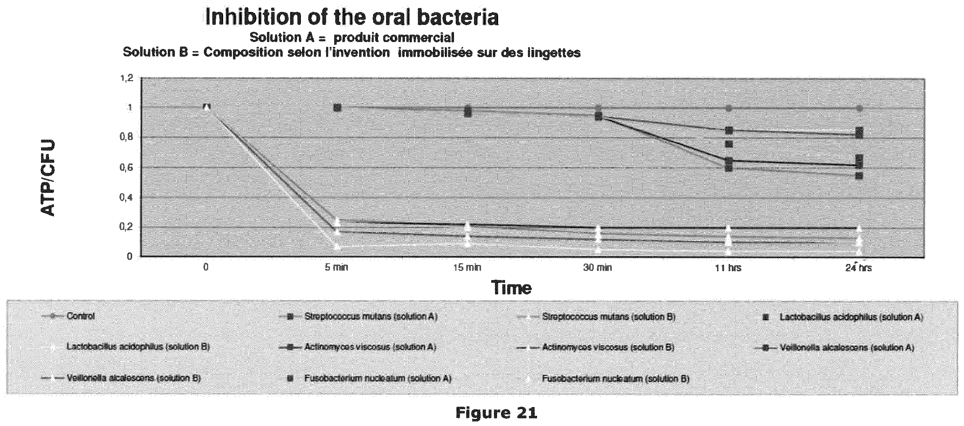

### Figure 21 : Action de la composition selon l'invention sur les biofilms

La figure 21 illustre l'action qu'a la composition selon l'invention (« solution B » ; points en forme de triangle) sur les différentes bactéries organisées en biofilm. Il est apparent que même à un temps de contact réduit (5 minutes), la population du biofilm est très significativement diminuée. Ceci n'est pas observé avec la composition commerciale de Chlorhexidine gluconate à 2% (« solution A » ; points en forme de carré).

La légende de la figure 21 :
- Points en forme de cercles : Contrôle
- Points en forme de carrés : Solution A correspondant au produit commercial (Chlorhexide gluconate à 2 %)
- Points en forme de triangles : Solution B correspondant à la composition selon l'invention, immobilisée sur des lingettes

De la courbe la plus proche du 0 ATP/CFU à 24 heures de traitement, et en remontant vers la courbe de contrôle définie par les points en forme de cercles, et correspondant à 1 ATP/CFU :
- Lactobacillus acidophilus en présence de la solution B
- Veillonella alcalescens en présence de la solution B
- Fusobacterium nucleatum en présence de la solution B
- Streptococcus mutans en présence de la solution B
- Actinomyces viscosus en présence de la solution B
- Streptococcus mutans en présence de la solution A
- Actinomyces viscosus en présence de la solution A
- Fusobacterium nucleatum en présence de la solution A
- Veillonella alcalescens en présence de la solution A
- Lactobacillus acidophilus en présence de la solution A

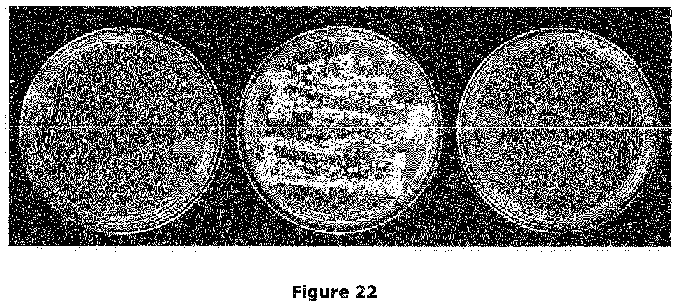

### Figure 22 : Action d'une solution d'I₂SCN⁻ sur des biofilms (résine). La résine est un matériau utilisé dans la confection de prothèses dentaires, obtenu par polymérisation de composés organiques.

La figure 22 est une photographie qui montre la stérilisation d'une languette de résine contaminée par *Candida albicans* ATCC 10231 après immersion dans une solution d'I₂SCN⁻. Le biofilm formé sur la résine a totalement été détruit après 30 min de contact à température ambiante. A gauche, on montre le Contrôle - qui est une languette de résine stérile, au milieu une languette contaminée par le *Candida albicans,* à droite une languette contaminée et désinfectée par une solution contenant 250 µM d'ions d'I₂SCN⁻

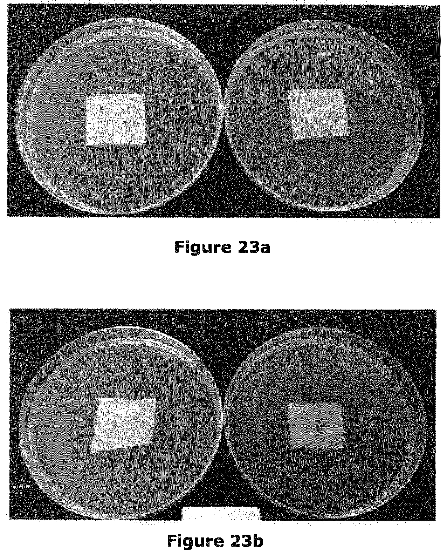

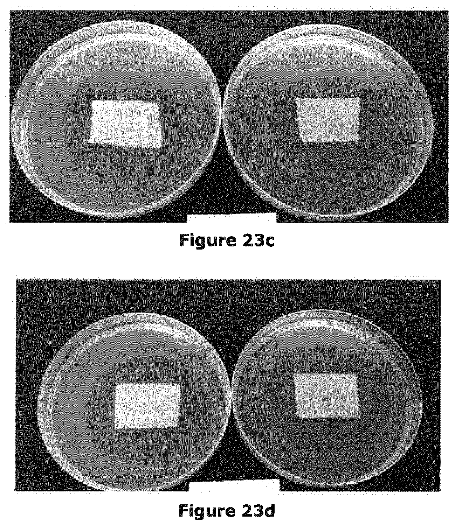

### Figures 23 : Action de la composition selon l'invention immobilisée sur un tissu.

Une composition selon l'invention a été préparée par mise en présence de 5,4 mM d'iodure de potassium (KI) de 1,2 mM de thiocyanate de potassium (KSCN) de 6,6 mM de peroxyde d'hydrogène (H₂O₂) en présence de 50 mg/L de lactoperoxydase (LP)(1000 unités ABTS par mg) dans un tampon citrate de sodium 100 mM pH 6,2 (figure 23b) ou un tampon phosphate 100 mM pH 7.4 (figure 23c) ou un tampon citrate de sodium 100 mM pH 6,2 et lyophilisé après préparation et reconstitué dans de l'eau (23d). Ces compositions ont été immobilisées sur un tissu et l'activité antibactérienne de ces tissus imbibés des compositions a été testée vis-à-vis d' *E. coli* (figure 23b, 23c, et 23d).

Les figures 23 illustrent l'action qu'a la composition selon l'invention sur *E. coli* (10⁹ cfu/ml). Il est apparent que après 24h d'incubation à 37°C de 100 µl d' *E. coli* à 10⁹ cfu/ml sur un milieu de culture sur boîte de petri, la composition immobilisée sur un tissu empêche le développement de la bactérie (halo apparent). Il est visible que la composition lyophilisée conserve une action bactéricide équivalente aux autres compositions non lyophilisée. Le tissu du « contrôle » a été imbibé d'eau stérile.

### EXEMPLES

### Exemple 1 : préparation de compositions selon l'invention

De manière générale, un certain nombre de compositions selon l'invention ont été préparées dans différentes conditions :
- de tampons/concentrations en tampon/pH ;
- de pH en l'absence de tampon (dans de l'eau) ;
- de rapports I⁻/SCN⁻,
- de concentration en peroxydase.

Les compositions selon l'invention sont préparées selon le protocole général décrit ci-après, ledit protocole étant accessible à l'homme du métier sans aucune autre explication.

Une première solution comprenant des ions iodure (I⁻) à une concentration molaire appropriée est préparée. Parallèlement une deuxième solution comprenant des ions thiocyanate (SCN⁻) à une concentration molaire appropriée est préparée. Parallèlement, une troisième solution de peroxyde d'hydrogène à une concentration molaire appropriée (soit la somme des deux concentrations molaires précédentes) est préparée.

Parallèlement, un « sachet de thé » comprenant de la lactoperoxydase (LP) est préparé.

Le « sachet de thé » est immergé dans de l'eau ou une solution aqueuse tamponnée.

Les deux premières solutions (comprenant respectivement les ions iodures et les ions thiocyanates) sont ajoutées à l'eau ou la solution aqueuse comprenant le « sachet de thé ».

La troisième solution (comprenant le peroxyde d'hydrogène) est ajoutée au mélange.

Au bout d'environ 60 secondes de présence à la fois des trois solutions (comprenant respectivement les ions iodures, les ions thiocyanates et l'H₂O₂) et de la lactoperoxydase (LP) est retirée au moyen du sachet de thé.

Après retrait de la lactoperoxydase (LP), plusieurs analyses peuvent être réalisées sur les produits de la réaction d'oxydation :
- Analyse RMN au ¹³C ;
- Mesure de l'activité oxydante des groupes -SH
- Mesure de l'activité oxydante des groupes -NH₂

Dans le cadre de la présente demande, la RMN au ¹³C a été utilisée pour identifier et quantifier les ions. En outre, elle a été utilisée pour confirmer qu'aucun ion hypothiocyanite (OSCN⁻) n'était détectable dans les compositions selon l'invention.

La présence du mélange ions I₂SCN⁻ et I(SCN)₂⁻ est prouvée par la présence d'un pic caractéristique à environ 49 à 50,5 ppm. L'absence des ions hypothiocyanites est prouvée par l'absence des pics à environ 127 à 128 ppm

Cette absence d'ions hypothiocyanites a également été mise en évidence par chromatographie ionique.
- Analyse de l'oxydation des fonctions SH et NH₂ ;
   L'analyse de l'oxydation des fonctions NH₂ est effectuée par oxydation de TMB (tetraméthylbenzydine).
   L'analyse de l'oxydation des fonctions SH est effectuée par oxydation du TNB (5-thio-2-nitrobenzoic acid) en DTNB (5,5'-dithiobis-(2-nitrobenzoic acid)).
- Test des compositions sur des micro-organismes

### Exemple 1.1 : influence du tampons/pH/concentrations en tampon

Le tableau récapitulatif des essais est présenté ci-dessous :

**Tableau 1**

| | | Oxydation | Oxydation | RMN (S13CN) | Position | Nouveau | RMN (intensité relative) | | |
|---|---|---|---|---|---|---|---|---|---|
| | | SH - µM/l | NH2 - µM/l | SCN- | OSCN- | Pic | SCN- | OSCN- | Nouveau Pic |
| **2,4 mM KSCN + 2,4 mM H₂O₂ + LP** | | | | | | | | | |
| 1 | Eau de ville | 374 | 0 | 133,48 | 127,71 | non | 320 | 320 | NA |
| 2 | Tampon phosphate -100 mM - pH 7,4 | - | | 133,48 | 127,71 | non | - | - | NA |

| **5,4 mM KI + 1,2 mM KSCN + 6,6 mM H₂O₂ + LP** | | | | | | | | | |
|---|---|---|---|---|---|---|---|---|---|
| 3 | Eau de ville | 442 | 504 | 133,48 | non | non | 160 | NA | NA |
| 4 | Tampon acétate Na - 500 mM - pH 4,4 | 864 | 1094 | non | non | **49,8** | NA | NA | 1425 |
| 5 | Tampon acétate Na - 100 mM - pH 4,4 | 829 | 1080 | non | non | **49,63** | NA | NA | 1650 |
| 6 | Tampon acétate Na - 10 mM - pH 4,4 | 827 | 1000 | non | non | **49,58** | NA | NA | 1475 |
| 7 | Tampon acétate Na - 1 mM - pH 4,4 | 365 | 164 | 133,48 | non | **49,62** | 110 | NA | 250 |
| 8 | Tampon acétate de NH₄ - 500 mM - pH 4,5 | 1191 | 1303 | non | non | **50,05** | NA | NA | 1800 |
| 9 | Tampon acétate de NH₄ - 100 mM - pH 4,5 | 964 | 1150 | non | non | **49,69** | NA | NA | 1700 |
| 10 | Tampon acétate de NH₄ - 10 mM - pH 4,5 | 962 | 1187 | non | non | **49,59** | NA | NA | 1550 |
| 11 | Tampon acétate de NH₄ - 1 mM - pH 4,5 | 576 | 539 | 133,48 | non | **49,6** | 150 | NA | 660 |
| 12 | Tampon acétate de Na - 500 mM - pH 5,6 | 1172 | 1074 | non | non | **50,51** | NA | NA | 1725 |
| 13 | Tampon acétate de Na - 100 mM - pH 5,6 | 983 | 1056 | non | non | **49,71** | NA | NA | 1800 |
| 14 | Tampon acétate de Na - 10 mM - pH 5,6 | 964 | 666 | 133,48 | non | **49,61** | 170 | NA | 200 |
| 15 | Tampon acétate de Na - 1 mM - pH 5,6 | 659 | 684 | 133,48 | non | **49,61** | 160 | NA | 290 |
| 16 | Tampon citrate - 500 mM - pH 6,2 | 1260 | 1375 | non | non | **51,11** | NA | NA | 1175 |
| 17 | Tampon citrate - 100 mM - pH 6,2 | 948 | 1000 | non | non | **49,97** | NA | NA | 1125 |
| 18 | Tampon citrate - 10 mM - pH 6,2 | 781 | 828 | non | non | **49,64** | NA | NA | 852 |
| 19 | Tampon phosphate - 500 mM - pH 7,4 | 1035 | 1552 | non | non | **51,16** | NA | NA | 1035 |
| 20 | Tampon phosphate -100 mM - pH 7,4 | 767 | 900 | non | non | **50,36** | NA | NA | 1650 |
| 21 | Tampon phosphate - 10 mM - pH 7,4 | 475 | 502 | 133,48 | non | non | 94 | NA | NA |
| 22 | Tampon phosphate - 1 mM - pH 7,4 | 271 | 284 | 133,48 | non | non | 205 | NA | NA |
| 23 | Tampon carbonate - 500 mM - pH 9,2 | 328 | 87 | 133,48 | non | non | 185 | NA | NA |
| 24 | Tampon carbonate - 100 mM - pH 9,2 | 248 | 106 | 133,48 | non | non | 225 | NA | NA |
| 25 | Tampon carbonate - 10 mM - pH 9,2 | 214 | 212 | 133,48 | non | non | 230 | NA | NA |

Il est tout d'abord précisé qu'aucun ion hypothiocyanite (OSCN⁻) n'est détecté pour toutes les compositions dans lesquelles les deux ions I⁻ et SCN⁻ ont été introduits (essais 3-25) alors que de tels ions se forment en l'absence de I⁻ (essais 1-2).

Comme expliqué en introduction de l'exemple 1, le pic aux alentours de 50 ppm en RMN est caractéristique des nouvelles espèces identifiées, à savoir I₂SCN⁻ et I(SCN)₂⁻. Par ailleurs son intensité est révélatrice de la quantité de nouvelles espèces formées.

Ce pic est observé lorsque le pH de la solution est compris entre 4,4 et 7,4. Lorsque le pH est inférieur à 4,4 , il y a hydrolyse du thiocyanate.

Lorsque le dosage en molécules oxydantes est élevé et que l'on voit l'apparition du pic à 49-50 ppm, on n'observe plus de pic lié au thiocyanate, ce qui montre bien sa participation à la réaction.

### Exemple 1.2 : influence du pH sur base eau (avec LP)

Le tableau récapitulatif des essais est présenté ci-dessous :

**Tableau 2**

| | | Oxydation SH-µM/l | Oxydation NH₂ -µM/l | RMN (S13CN) SCN- | Position OSCN- | Nouveau Pic | RMN (intensité relative) | | |
|---|---|---|---|---|---|---|---|---|---|
| | | | | | | | SCN- | OSCN- | Nouveau Pic |
| **5,4 mM KI + 1,2 mM KSCN** + **6,6 mM H₂O₂ + LP** | | | | | | | | | |
| 26 | Eau - pH 4,4 | 1130 | 1622 | non | non | **49,61** | NA | NA | 1300 |
| 27 | Eau - pH 5,5 | 1010 | 821 | non | non | **49,62** | NA | NA | 740 |
| 28 | Eau - pH 6,5 | 644 | 1098 | 133,48 | non | non | 120 | NA | NA |
| 29 | Eau - pH 7,5 | 505 | 567 | 133,48 | non | non | 210 | NA | NA |

Ici encore, en présence d'ion iodure, aucun ion hypothiocyanate (OSCN⁻) n'est détecté, quel que soit le composé.

On constate que lorsque la solution aqueuse non tamponnée est à pH inférieur à 6,5, on détecte le nouveau pic caractérisé par un déplacement en RMN (49,6 ppm), corrélé à une capacité accrue à oxyder les groupes SH et NH₂.

Le nouveau pic est observé à des pH acides : 5,5 et 4,4, avec une intensité quasi-double à pH 4,4.

En revanche, à des pH supérieurs, le nouveau pic n'est pas observé.

### Exemple 1.3 : influence du rapport I⁻/SCN⁻

Le tableau récapitulatif des essais est présenté ci-dessous :

**Tableau 3**

| | | RMN (S13CN) SCN⁻ | Position OSCN⁻ | Nouveau Pic | Autres Pics | RMN (intensité relative) | | | | |
|---|---|---|---|---|---|---|---|---|---|---|
| | | | | | | SCN⁻ | OSCN- | Nouveau Pic | Autres Pics | |
| 30 | **9:1** 5,4 mM KI + 0,6 mM KSCN + 6 mM H₂O₂ T Na acétate pH4,4 0,1M | non | non | 49,6 | non | NA | NA | 740 | NA | NA |
| 31 | **1:1** 5,4 mM KI + 5,4 mM KSCN + 10,8 mM H₂O₂ T Na acétate pH4,4 0,1M | 133,47 | non | 49,65 | 124,63 /111,94 | 560 | NA | 190 | 800 | 130 |
| 32 | **0,54: 1** 5,4 mM KI + 10 mM KSCN + 15,4 mM H₂O₂ T Na acétate pH4,4 0,1M | 133,48 | non | non | 124,63/111,94 | 1400 | NA | NA | 1750 | 150 |
| 33 | **1:1** 1,2 mM KI + 1,2 mM KSCN + 2,4 mM H₂O₂ T Na acétate pH4,4 0,1M | 133,48 | non | non | 124,63/111,93 | 75 | NA | NA | 125 | 105 |
| 34 | **0,27** : **1** 1,2 mM KI + 5,4 mM KSCN + 6,6 mM H₂O₂ T Na acétate pH4,4 0,1M | 133,48 | non | non | 124,63 | 825 | NA | NA | 750 | NA |
| 35 | **1:2** 5,4 mM KI + 2,7 mM KSCN + 8,1 mM H₂O₂ T Na acétate pH4,4 0,1M | 133,48 | non | 49,62 | non | 100 | NA | 950 | NA | NA |
| 36 | **4,5** 5,4 mM KI + 1,2 mM KSCN + 6,6 mM H₂O₂ + T acétate d'ammonium 100 mM pH 4,5 | non | non | 49,69 | non | NA | NA | 1700 | NA | NA |

Ici encore, en présence d'ion iodure, aucun ion hypothiocyanate (OSCN⁻) n'est détecté, quel que soit le composé.

Le nouveau pic est observé à des rapports I⁻/SCN⁻ compris entre 9/1 et strictement supérieur à 1/1.

En revanche, aucun pic n'est observé à des rapports I⁻/SCN⁻ compris entre 0,27/1 et 1/1.

### Exemple 2 : Efficacité d'une composition selon l'invention

Une composition selon l'invention a été préparée par mise en présence d'iodure de potassium (KI) 5,4 mM de 1,2 mM de thiocyanate de potassium (KSCN) de 6,6 mM de peroxyde d'hydrogène (H₂O₂) dans un tampon acétate d'ammonium 100 Mm, pH 4,5 en présence de 50 mg/L de lactoperoxydase (LP) (1000 unités ABTS par mg) selon le protocole décrit à l'exemple 1.

Ce composé a été comparé aux composés de l'art antérieur fabriqué par mise en présence :
- soit d'iodure de potassium (KI), de lactoperoxydase (LP) et de peroxyde d'hydrogène (H₂O₂) ;
- soit de thiocyanate de potassium (KSCN), de lactoperoxydase (LP) et de peroxyde d'hydrogène (H₂O₂).

Les résultats sont donnés dans le tableau ci-dessous :

**Tableau 4**

| | **KSCN + KI** | **KSCN** | **KI** |
|---|---|---|---|
| *Colletotrichum lindemuthanium* | ++++ | - | - |
| *Fusarium avenaceum* | +++ | - | |
| *Septoria tritici* | + | - | - |
| *Verticillium dahliae* | ++++ | - | - |
| *Phytophthora infestons* | +++ | - | - |
| *Pythium ultimum* | ++++ | - | - |
| *Colletotrichum musae* | ++++ | 0 | 0 |
| *Pencillium italicum* | ++++ | 0 | ++++ |
| *Penicillium digitatum* | ++++ | + | ++++ |
| *Botrytis cinerea* | ++++ | - | - |
| *Penicillium expansum* | ++++ | - | - |
| *Pectobacterium atrosepticum* | ++++ | + | + |
| *Pseudomonas syringae pv syringae* | ++++ | + | 0 |
| *Pectobacterium atrosepticum* | ++++ | + | 0 |
| *Erwynia amylovora* | ++++ | + | 0 |
| *Pseudomona syringa pv. tomato* | ++++ | +++ | + |
| *Clavibacter michiganensis subsp. michiganensis* | ++++ | ++++ | +++ |
| *Kocuria rhizolia* | ++++ | + | + |
| *Staphylococcus aureus* | ++++ | ⁺+ | ++ |
| *Enterobacter gergoviae* | ++++ | + | + |
| *Escherichia coli* | ++++ | + | + |
| *Klesiella pneumoniae* | ++++ | + | + |
| *Pseudomonas aeruginosa* | ++++ | + | + |
| *Pseudomonas fluorescent* | ++++ | + | + |
| *Pseudomonas putita* | ++++ | + | + |
| *Aspergillus niger* | ++++ | - | + |
| *Penicillium pinophilum* | ++++ | + | ++++ |
| *Candida albicans* | ++++ | + | ++ |
| *Xylella fastidiosa subsp. fastidiosa* | ++++ | - | - |
| *Xylella fastidiosa subsp. multiplex* | ++++ | - | - |
| *Xylella fastidiosa subsp. pauca* | ++++ | - | - |
| **%** ***d'inhibition** :* | | | |
| ***++++ : 79-100 %*** | | | |
| ***+++ : 60-78 %*** | | | |
| ***++ :* 41-59 %** | | | |
| ***+ : 0-40* %** | | | |
| ***0** : **pas d'inhibition*** | | | |
| ***-** : **non testé*** | | | |

On observe qu'une compositon selon l'invention a une activité souvent très supérieure sur l'ensemble des micro-organismes testés.

### Exemple 3 : Pouvoir oxydant d'une composition selon l'invention

Une composition selon l'invention a été préparée par mise en présence d'iodure de potassium (KI) 5,4 mM de 1,2 mM de thiocyanate de potassium (KSCN) de 6,6 mM de peroxyde d'hydrogène (H₂O₂) dans un tampon acétate d'ammonium 100 mM, pH 4,5 en présence de 50 mg/L de lactoperoxydase (LP) (1000 unités ABTS par mg) selon le protocole décrit à l'exemple 1.

Le temps de mise en contact des différentes solutions a été fixé à 1 minute.

Le pouvoir oxydant a ensuite été mesuré, sur les fonctions SH par la méthode de l'oxydation du TNB (5-thio-2-nitobenzoic acid) en DTNB (5,5'-dithiobis-(2-nitrobenzoic acid)), au bout des temps de conservation suivants : 1, 3, 5, 10, 15, 20, 30, 60 et 120 minutes.

Le temps T=0 correspond au moment où la lactoperoxydase (LP) est retirée.

Les résultats sont donnés dans le tableau ci-dessous :

**Tableau 5**

| Temps (minutes) | Oxydation -SH |
|---|---|
| 0 | 516,54 |
| 3 | 567,64 |
| 5 | 586,4 |
| 10 | 586,7 |
| 15 | 587,02 |
| 20 | 584,68 |
| 30 | 581,78 |
| 60 | 701,58 |
| 120 | 684,7 |

Il est remarqué que l'activité oxydante augmente avec le temps de conservation.

De plus, après 60 minutes, le pouvoir oxydant est stable.

### Exemple 4 : Stabilité d'une composition selon l'invention

Une composition selon l'invention a été préparée par mise en présence d'iodure de potassium (KI) 5,4 mM de 1,2 mM de thiocyanate de potassium (KSCN) de 6,6 mM de peroxyde d'hydrogène (H₂O₂) dans un tampon acétate d'ammonium 100 mM, pH 4,5 en présence de 50 mg/L de lactoperoxydase (LP) (1000 unités ABTS par mg) selon le protocole décrit à l'exemple 1.

Cette composition a été répartie dans 6 flacons. Les flacons sont ensuite ouverts (1 par mois) et l'activité bactéricide sur *candida albicans* est testée.

Le graphique illustrant la variation du taux de survie des *candida albicans* en fonction du temps, évaluée durant une période de 6 mois, est présenté en figure 5

Il est observé qu'aucune diminuation mesurable de l'activité n'est mise en évidence durant la période de 6 mois.

### Exemple 5 : Essai d'un temps court de mise en contact avec candida albicans (5 minutes)

Une composition selon l'invention a été préparée par mise en présence d'iodure de potassium (KI) 5,4 mM de 1,2 mM de thiocyanate de potassium (KSCN) de 6,6 mM de peroxyde d'hydrogène (H₂O₂) dans un tampon acétate d'ammonium 100 Mm, pH 4,5 en présence de 50 mg/L de lactoperoxydase (LP) (1000 unités ABTS par mg) selon le protocole décrit à l'exemple 1.

L'activité bactéricide sur *candida albicans* a été testée, pour les temps de mise en contact avec candida albicans suivants : 5 minutes ou 30 minutes.

Après un temps de mise en contact de 5 minutes ou 30 minutes avec la composition (ou le contrôle), un ensemencement a lieu sur un milieu de culture.

Les résultats de ces tests sont illustrés en figure 6 (photographies des milieux de culture après la mise en contact versus contrôle) : que le temps de contact soit de 30 minutes (partie gauche de la figure) ou de 5 minutes (partie droite de la figure), le résultat visuel est le même : aucune colonie de *candida albicans* ne résiste à la composition selon l'invention.

### Exemple 6 : Efficacité avec une concentration d'oxydants diminuée

Une composition selon l'invention a été préparée par mise en présence d'iodure de potassium (KI) 5,4 mM de 1,2 mM de thiocyanate de potassium (KSCN) de 6,6 mM de peroxyde d'hydrogène (H₂O₂) dans un tampon acétate d'ammonium 100 mM, pH 4,5 en présence de 50 mg/L de lactoperoxydase (LP) (1000 unités ABTS par mg) selon le protocole décrit à l'exemple 1.

Cette composition a été répartie dans 5 flacons, selon différentes dilutions, dans l'ordre décroissant : 755 µm, 252 µm, 75 µm, 25 µm. Une solution de contôle a également été réalisée.

Après un temps de mise en contact de 5 minutes avec les 6 différentes composition (5 dilutions et le contrôle), un ensemencement a lieu sur un milieu de culture.

Les résultats sont présentés dans la figure 7 : même à une concentration de 25 µm, il est observé que le nombre de colonies formées est fortement diminué, par rapport au contrôle. En outre, jusqu'à 252 µm, l'activité n'est pas diminuée.

### Exemple 7 : Sélectivité d'une composition selon l'invention streptococcus mutans / streptococcus salivarius

Une composition selon l'invention a été préparée par mise en présence d'iodure de potassium (KI) 5,4 mM de 1,2 mM de thiocyanate de potassium (KSCN) de 6,6 mM de peroxyde d'hydrogène (H₂O₂) dans un tampon acétate d'ammonium 100 mM, pH 4,5 en présence de 50 mg/L de lactoperoxydase (LP) (1000 unités ABTS par mg) selon le protocole décrit à l'exemple 1.

La composition est testée quant à son effet bactéricide sur *streptococcus mutans* (bactérie cariogène) et sur *streptococcus salivarius* (bactérie commensale).

Les résultats sont présentés dans la figure 8 : la composition selon l'invention a un effet bactéricide sur *streptococcus mutans* (partie gauche de la figure) et n'a pas d'effet sur *streptococcus salivarius* (partie droite de la figure).

### Exemple 8 : Activité vis-à-vis des biofilms

Une compositon selon l'invention selon le protocole décrit à l'exemple 1.

Cette composition a été diluée 3 fois et présente une concentration en ions I₂SCN⁻ de 250 µM. Cette compostion est appelée « solution B ».

Cette composition a été mise en contact avec différentes bactéries organisées en biofilms, versus contrôle et versus autre composition du commerce qui est de la Chlorexhidine gluconate 2,0% (« solution A »).

Les compositions ont été mises en contact avec le biofilm des différentes bactéries, pendant des durées variables : 5 minutes, 15 minutes, 30 minutes, 11 heures et 24 heures. Cette mise en contact se fait par immersion des languettes contenant le biofilm bactérien dans une solution de la composition ou soit dans une solution de chlorexhidine gluconate 2% ou comme contrôle dans une solution aqueuse (voir ci-après les détails de la technique appliqée pour le *Candida albicans)*

L'activité vis-à-vis des bactéries suivantes a été testée : *lactobacillus acidophilus, veillonella alcalescens, streptococcus mutans, actinomyces viscosus, et fusobacterium nucleatum.*

Les résultats sont présentés dans la figure 21 : la composition selon l'invention est active sur toutes les bactéries testées, dès 5 minutes de contact, contrairement à la solution de Chlorexhidine gluconate 2% qui a une action moins efficace, et seulement à partir de 11 heures de mise en contact. Le contrôle n'a, évidemment, aucune action.

### • Biofilms candida abicans

### Protocole de fixation des biofilms de Candida albicans sur résine et titane.

### A) Résine : Matériau utilisé dans la confection de prothèses dentaires, obtenu par polymérisation de composés organiques

Les morceaux de résine sont conservés dans de l'azide de sodium (0,5 g / 500 mL) pour leur désinfection. Cette manipulation se fait dans des conditions stériles.

Transférer 3 morceaux de résine dans un potiquet.

Laver 3x dans 60 mL d'H₂O pendant 5 min avec agitation.

Rincer une dernière fois avec 60 mL de Sabouraud liquide pendant 5 min avec agitation.

Transférer chaque morceau de résine dans un tube à fond rond de 10 mL.

Préparer une suspension de *Candida albicans* de 10⁶ bl/mL dans 10 mL de milieu Sabouraud.

Préparer les 3 tubes réactionnels comme indiqué dans le tableau ci-dessous.

| Préparation des biofilms sur résine (composition des tubes au J₁). Sabouraud liquide (mL) | | Suspension de C.a. (mL) |
|---|---|---|
| Contrôle - | 4 | |
| Contrôle + | 3,6 | 0,4 |
| Essai | 3,6 | 0,4 |

Incuber pendant 24 à 48 h dans un Rotator™ (3 tours/min).
• Après incubation, à partir de chacun des 3 tubes, transférer 1 mL de surnageant dans une cuvette. Mesurer l'absorbance à 600 nm.
• Aspirer les milieux de culture dans les 3 tubes.
• Laver 3x dans du tampon phosphate.
• Préparer une solution de tampon phosphate contenant du glucose (2 g / 100 mL).
• A partir de ce tampon, préparer une solution d'I₂SCN⁻.
• Préparer les 3 tubes réactionnels comme indiqué dans le tableau ci-dessous.

| Préparation des biofilms sur résine (composition des tubes au J₂). Tampon phosphate + glucose (mL) | | I₂SCN⁻ (mL) |
|---|---|---|
| Contrôle - | 4 | - |
| Contrôle + | 4 | - |
| Essai | - | 4 |

• Incuber 30 min.
• Ensemencer chaque face des languettes de résine successivement sur 4 boîtes de Petri.
• Incuber pendant 24 à 48h à 37°C.

### B) Titane

Cette manipulation se fait dans des conditions stériles.

Peser 500 mg de titane dans 3 tubes différents.

Préparer une suspension de *Candida albicans* de 10⁶ bl/mL dans 10 ml de milieu Sabouraud.

Préparer les 3 tubes réactionnels comme indiqué dans le tableau ci-dessous.

| Préparation des biofilms sur titane (composition des tubes au J₁). Sabouraud liquide (mL) | | Suspension de C.a. (mL) |
|---|---|---|
| Contrôle - | 4 | - |
| Contrôle + | 3,6 | 0,4 |
| Essai | 3,6 | 0,4 |

Incuber 24 à 48h dans un Rotator™ (3 tours/min).
• Après incubation, laisser sédimenter exactement 10 min.
• A partir de chacun des 3 tubes, transférer 1 mL de surnageant dans une cuvette. Mesurer l'absorbance à 600 nm.
• Aspirer les milieux de culture dans les 3 tubes.
• Laver 3 fois dans du tampon phosphate.
• Préparer une solution d'I₂SCN⁻.
• Préparer une solution de tampon phosphate contenant du glucose (2 g / 100 mL).
• Préparer les 3 tubes réactionnels comme indiqué dans le tableau ci-dessous.

| Préparation des biofilms sur titane (composition des tubes au J₂). Tampon phosphate + glucose (mL) | | I₂SCN⁻ (mL) |
|---|---|---|
| Contrôle - | 4 | - |
| Contrôle + | 4 | - |
| Essai | - | 4 |

Incuber 30 min.
• Aspirer le surnageant

### Dosage au MTT (*)

Les blastoconidies vivantes transforment le MTT-tetrazolium en MTT-formazan qui absorbe à 570 nm (Levitz & Diamond, 1985).

Les différentes étapes de la procédure sont détaillées ci-dessous :
• Préparer une suspension de *Candida albicans* de 10⁶ bl/ml dans 10 ml de tampon phosphate contenant du glucose (2 g / 100 mL)
• (*) MTT : Bromure de 3-(4,5-dimethylthiazol-2-yl)-2,5-diphenyltetrazolium
• Préparer les 4 tubes réactionnels comme indiqué dans le tableau ci-dessous.

| Préparation des tubes pour le dosage au MTT. Blanc | | Contrôle - | Contrôle + | Essai |
|---|---|---|---|---|
| Suspension de *C.a.* (mL) | - | - | - | - |
| MTT (mL) | 1,5 | 1,5 | 1,5 | 1,5 |
| Tampon phosphate + glucose (mL) | 3 | 3 | 3 | 3 |

A la Figure 22 sont représentées :
Contrôle - (à gauche): languette de résine stérile.
Contrôle + (au milieu): languette contaminée.
Essai (à droite): languette contaminée et désinfectée par la solutionA

On observe la stérilisation d'une languette de résine contaminée par *Candida albicans* ATCC 10231 après immersion dans une solution contenant 250µM d'ions I₂SCN⁻. Le biofilm formé sur la résine a totalement été détruit après 30 min de contact à température ambiante.

L'action d'une solution d'I₂SCN⁻ sur des biofilms sur (titane) est mesurée par dosage des biofilms à Candida au MTT.

La solution contenant 250 µM d'ions I₂SCN⁻ permet de détruire ∼70% des biofilms formés pendant 24 à 48h.

Les résultats obtenus sont rassemblés ci-dessous :

| | | |
|---|---|---|
| Contrôle - 0,003 | Contrôle + 0,949 | Essai 0,319 |

### Exemple 9 : Stabilité et activité d'une composition selon l'invention pour l'application aux problèmes de contamination des peintures et des résines

Une composition selon l'invention a été préparée par mise en présence d'iodure de potassium (KI) 5,4 mM de 1,2 mM de thiocyanate de potassium (KSCN) de 6,6 mM de peroxyde d'hydrogène (H₂O₂) dans un tampon acétate d'ammonium 100 mM, pH 4,5 en présence de 50 mg/L de lactoperoxydase (LP) (1000 unités ABTS par mg) selon le protocole décrit à l'exemple 1.

Les échantillons de peintures et de résines ont été contaminés par plusieurs séries de micro-organismes tels que des bactéries, des levures et des moisissures et les mélanges d'entre eux.

Les tests ont été effectués en additionnant la composition selon l'invention dès le départ. Après une attente de 24 heures, les échantillons de résine et de peinture ont été inoculés avec une suspension d'un mélange de micro-organismes de façon à atteindre un taux de contamination de 1,000,000 CFU/ml. Les mélanges des micro-organismes ont été composés de bactéries, levures et moisissures telles que :
- bacteries : *Pseudomonas fluorescent ATCC 9721, Pseudomonas aeruginosa ATCC 10145, Bacillus substilis ATCC 6984, Proteus vulgaris ATCC 9920 ;*
- levures : *Candida tropicalis ATCC 750, Kluyveromyces fragilis ATCC 8554, Candida pseudotropicalis ATCC 4135 ;*
- moisissures : *Aspergillus niger ATCC 9642, Aspergillus flavus ATCC 9643, Pénicillium pinophilum ATCC 9644.*

Après des temps de contact de 1, 2 et 7 jours, les analyses microbiologiques ont été effectués. Elles sont résumées dans le tableau 6 ci-dessous :

**Tableau 6**

| **Mélange A: 1,000,000 CFU/ml** | **1 jours** | **2 jours** | **7 jours** |
|---|---|---|---|
| *Pseudomonas fluorescent* | ++++ | ++++ | ++++ |
| *Pseudomonas aeruginasa* | ++++ | ++++ | ++++ |
| *Candida tropicalis* | ++++ | ++++ | ++++ |
| *Aspergillus niger* | ++++ | ++++ | ++++ |
| | | | |

| **Mélange B: 1,000,000 CFU/ml** | | | |
|---|---|---|---|
| *Pseudomonas aeruginasa* | ++++ | ++++ | ++++ |
| *Kluyveromyces fragilis* | ++++ | ++++ | ++++ |
| *Penicillium pinophilum* | ++++ | ++++ | ++++ |
| | | | |

| *% d'hibition* | | | |
|---|---|---|---|
| ++++ | 79-100 % | | |
| +++ | 60-78% | | |
| ++ | 41-59 % | | |
| + | 0-40% | | |
| 0 | Pas d'inhibition | | |

On remarque qu'il n'y a pas de contamination quel que soit l'échantillon. Ceci confirme que la composition selon l'invention a une activité empêchant la croissance des micro-organismes dans les peintures et les résines.

### Exemple 10 : Activité d'une composition selon l'invention pour les problèmes de contamination des sols, matériaux de production et des instruments dentaires, chirurgicaux, etc.

Dans les processus industriels, on applique un nettoyage que l'on nomme CIP (Cleaning In Place) et qui consiste à faire passer des désinfectants à haute température après l'utilisation d'équipements.

Il en est de même pour les instruments utilisés dans les cabinets dentaires, dans les hôpitaux que l'on nettoie après leur utilisation par une stérilisation à l'étuve à haute température.

Une composition selon l'invention a été préparée par mise en présence d'iodure de potassium (KI) 5,4 mM de 1,2 mM de thiocyanate de potassium (KSCN) de 6,6 mM de peroxyde d'hydrogène (H₂O₂) dans un tampon acétate d'ammonium 100 mM, pH 4,5 en présence de 50 mg/L de lactoperoxydase (LP) (1000 unités ABTS par mg) selon le protocole décrit à l'exemple 1.

Il a été démontré que cette composition était capable d'éliminer les micro-organismes responsables des contaminations de ces équipements industriels et autres et avait l'avantage de pouvoir être utilisée à température ambiante.

### Exemple 11 : Activité d'une composition selon l'invention pour les problèmes de contamination lors de la cicatrisation de blessures chez l'homme et l'animal

La résistance des micro-organismes aux antibiotiques est un problème croissant et complique gravement la cicatrisation des plaies occasionnées par une blessure ou une brûlure sur la peau. Ces micro-organismes sont capables d'accroître le processus inflammatoire.

Une compositon selon l'invention a été préparée selon le protocole décrit à l'exemple 1 et a été diluée jusqu'à obtenir une composition comprenant 250µM de I₂SCN⁻.

Cette composition a été testée sur des micro-organismes résistants aux antibiotiques actuels.

Plus précisément, l'utilisation de tissus imbibés a montré la possibilité lorsqu'ils étaient appliqués sur la plaie, d'avoir une activité antibactérienne contre des espèces de bactéries résistantes à différents antibiotiques.

Les résultats décrits dans le tableau ci-dessous montrent que la composition selon l'invention qui a été préparée selon le protocole décrit à l'exemple 1 est très efficace contre ces micro-organismes même sa concentration en ions I₂SCN⁻ est de 250µM.

**Tableau 7**

| **Bacteries** | | **1 jours** | **2 jours** | **7 jours** |
|---|---|---|---|---|
| Burkholderia multivorans | **multiresistant** | ++++ | ++++ | ++++ |
| Pseudomonas aeruginosa | **multiresistant** | ++++ | ++++ | ++++ |
| Stenotrophomonas maltophilia | **multiresistant** | ++++ | ++++ | ++++ |
| Pandoraea apista | **multiresistant** | ++++ | ++++ | ++++ |
| Achromobacter denitrificans | **multiresistant** | ++++ | ++++ | ++++ |
| Staphylococcus aureus | **multiresistant** | ++++ | ++++ | ++++ |
| Enterococcus faecium | **multiresistant** | ++++ | ++++ | ++++ |
| Enterococcus faecalis | **multiresistant** | ++++ | ++++ | ++++ |
| | | | | |

| **Levures** | | | | |
|---|---|---|---|---|
| malassezia pachydermatis | **multiresistant** | ++++ | ++++ | ++++ |

### Exemple 12 : Activité d'une composition selon l'invention contre les micro-organismes responsables des maladies respiratoires chez les chevaux et chez l'homme

Une composition selon l'invention a été préparée selon le protocole décrit à l'exemple 1 et a été testée sur des micro-organismes responsables de maladies respiratoires chez les chevaux et chez l'homme.

Les courbes de croissance entre la phase exponentielle et la phase stationnaire de croissance, nous ont permis de choisir les conditions idéales qui correspondaient à une concentration de 1,000,000 spores/ml. Un composition selon l'invention a été préparée selon le protocole décrit à l'example 1 et a montré une efficacité contre les micro-organismes responsables des maladies respiratoires chez le cheval.

Les micro-organismes étaient les suivants: *Rhodococcus equi ATCC 25729 - Streptococcus equi subsp equi ATCC 53185 et de Streptococcus equi subsp zooepidemicus ATCC 43079.* Ils sont responsables des maladies respiratoires chez les chevaux.

Compte tenu du temps de croissance de ces micro-organismes, les essais ont été effectués après une croissance de 48 heures et de 120 heures du micro-organisme.

Dans tous les mélanges, le peroxyde d'hydrogène est consommé dans son intégralité. Le thiocyanate et l'iode sont consommés en proportions identiques.

La composition préparée selon le protocole décrit à l'exemple 1 a montré une efficacité sur l'inhibition des micro-organismes après un temps de contact de 5 minutes. 4 concentrations en I₂SCN⁻ de la composition d'ions ont été utilisées à différentes dilutions. Chaque donnée représente les résultats de 3 expériences effectuées indépendamment.

Les pourcentages d'inhibition in vitro des micro-organismes ont été mesurés après un temps de contact de 5 minutes.

Des contrôles ont été faits avec des solutions sans enzyme avec uniquement les substrats 5,4 mM KI + 2,2 mM KSCN d'une part et avec 6,6 mM d'H₂O₂. Ces solutions ont montré une absence d'efficacité sur les micro-organismes (voir tableau 8 ci-dessous).

**Tableau 8**

| | | **Avant réaction enzymatique** | | | | | | | |
|---|---|---|---|---|---|---|---|---|---|
| | | | | | | | | ***Streptococcus equi*** | ***Streptococcus equl*** |
| **LPO** | | **KI/KSCN/H2O2** | **Dilution** | *Rhodococcus equi* | *Rhodococcus equi* | *Streptococcus equi* | *Streptococcus equi* | *zooepidemicus* | *zooepidemicus* |
| **U/ml** | | **mM/L** | | **% inhibition** | **% inhibition** | **% Inhibition** | **% inhibition** | **% Inhibition** | **% inhibition** |
| | | | | **à 48h** | **à 120 h** | **à 48 h** | **à 120 h** | **à 48 h** | **à 120 h** |
| | 50 | **5,4/1,2/6,6** | **1/3-** | **86** | **88** | **82** | **87** | **92** | **97** |
| | | | **1/5-** | **82** | **84** | **80** | **86** | **92** | **94** |
| | | | **1/10-** | **81** | **83** | **60** | **62** | **90** | **94** |
| | | **3,6/0,8/4,4** | **1/3-** | **86** | **88** | **65** | **69** | **95** | **98** |
| | | | **1/5-** | **86** | **88** | **60** | **61** | **74** | **77** |
| | | | **1/10-** | **63** | **70** | **65** | **69** | **44** | **48** |
| | | **2,7/0,6/3,3** | **1/3-** | **82** | **84** | **60** | **63** | **79** | **81** |
| | | | **1/5-** | **81** | **81** | **68** | **72** | **10** | **13** |
| | | | **1/10-** | **21** | **24** | **64** | | **67 10** | **13** |
| | | **0,78/0,34/1,1** | **1/3-** | **85** | **90** | **30** | **35** | **14** | **19** |
| | | **Effets des substrats** | **1/3-** | **0** | **0** | **4** | **6** | **4** | **7** |
| | | **sans présence d'enzyme** | **1/3-** | **0** | **0** | **0** | **0** | **0** | **0** |

Une seconde série d'essai a été réalisée dans les mêmes conditions avec d'autres micro-organismes responsables des maladies respiratoires chez l'homme et qui ont été détectés dans les cas de mucoviscidose. : Burkholderia cepacia tobramycin resistant (ATCC BAA-245), Pseudomonas aeruginosa mucoïd, Staphylococcus aureus résistant au methycillin et à l'oxacillin (ATCC 43300).

**Tableau 9**

| | | **Avant réaction enzymatique** | | **HOMME** | | |
|---|---|---|---|---|---|---|
| **LPO** | | **KI/KSCN/H2O2** | **Dilution** | ***Burkholderia cepacia*** | ***Pseudomonas aeruginosa* (mucoid)** | ***Staphylococcus aureus* (MRSA) methycillin and oxacillin resistant)** |
| **U/ml** | | **mM/L** | | **Temps de contact 5 minutes % inhibition** | **Temps de contact 5 minutes % inhibition** | **Temps de contact 5 minutes % inhibition** |
| | **50** | **5,4/1,2/6,6** | **1/1-** | **100** | **100** | **100** |
| | | | **1/3-** | **100** | **100** | **100** |
| | | | **1/5-** | **78** | **57** | **56** |
| | | | **1/10-** | **32** | **38** | **36** |
| | | **Effets des substrats** | **1/1-** | **0** | **0** | **0** |
| | | **sans présence d'enzyme** | **1/3-** | **0** | **0** | **0** |

En résumé, une forte activité anti-micro-organismes a été mise en évidence.

### Exemple 13 : Activité d'une composition selon l'invention contre les micro-organismes responsables de la détérioration des plantes, fruits et légumes et autres plantes récoltées notamment chez les bananes

Une composition selon l'invention a été préparée par mise en présence d'iodure de potassium (KI) 5,4 mM de 1,2 mM de thiocyanate de potassium (KSCN) de 6,6 mM de peroxyde d'hydrogène (H₂O₂) dans un tampon acétate d'ammonium 100 mM, pH 4,5 en présence de 50 mg/L de lactoperoxydase (LP) (1000 unités ABTS par mg) selon le protocole décrit à l'exemple 1.

Cette compositions a été testée sur des bananes contaminées notamment par des champignons causant des lésions d'anthracnose et des pourritures de la couronne.

En plongeant les bananes dans la composition préparée selon le protocole décrit à l'exemple 1, on a démontré que la dite composition avait une grande efficacité contre les infections dues au *Colletotricum musae, au Fusarium monoliforme* et au *fusarium oxysporum*

De plus, il a été démontré que la composition préparée selon le protocole décrit à l'exemple 1, était plus active contre les champignons comparés aux fongicides classiques qui sont toxiques et qui polluent l'environnement.

### Exemple 14 : Activité sur Xylella fastidiosa

Une composition selon l'invention a été préparée par mise en présence d'iodure de potassium (KI) 5,4 mM de 1,2 mM de thiocyanate de potassium (KSCN) de 6,6 mM de peroxyde d'hydrogène (H₂O₂) dans un tampon citrate-phosphate 100 Mm, pH 6,9 en présence de 50 mg/L de lactoperoxydase (LP) (1000 unités ABTS par mg) selon le protocole décrit à l'exemple 1.

La composition est testée quant à son effet bactéricide sur *Xylella fastidiosa subsp. fastidiosa, Xylella fastidiosa subsp. multiplex et Xylella fastidiosa subsp. Pauca* selon le protocole décrit ci-après.

Les résultats sont présentés dans les figures 9, 10 et 11 : la composition selon l'invention a un effet bactéricide à la fois sur *Xylella fastidiosa subsp. fastidiosa, Xylella fastidiosa subsp. multiplex et Xylella fastidiosa subsp. pauca.*

L'inoculum est préparé par 2 repiquages successifs (chaque repiquage à 26°C pendant 10 jours) sur un milieu comprenant un mélange des 3 solutions A, B et C décrites ci-dessous ; le mélange de A et B étant stérilisé à l'autoclave avant ajout de la solution C stérilisée par filtration.
- Solution A: 500 ml d'eau distillée (Aq.Dest) (50°C) + 10 gr ACES Sigma A9758-25G
- Solution B: 440 ml d'eau distillée (Aq.Dest) + 40 ml 1,0 N KOH (1N KOH : 2,24 gr solution 40ml d'eau distillée.)

A la solution B sont ensuite ajoutés 2gr de charcoal (Active Charcoal Sigma C-4386) 10 gr de Yeast extract oxoid et 17 gr d'Agar.

Le mélange des deux solutions est effectué puis stérilisé à l'autoclave.
- Solution C: cysteine HCl 0,4 gr + ferric pyrophosphate 0,25 gr solution 20 ml d'eau distillée (Aq.Dest) Stérilisation à froid (filtre 0,2µ)

5 ml de PBS stérile sont ensuite ajoutés dans une boite comprenat une souche de xylella ; la boite est raclée avec un rateau stérile et les 5 ml sont pipetés dans un flacon stérile.

La DO650 est ajustée à 1 (DO650 de 1=104 cfu/ml réf. Shi et al, 2007, Appl. Environ. Microbiol.,73 (21)) avec du PBS stérile.
- dans un tube falcon 15 ml, ajouter :

Témoin : 1 ml inoculum, 1 ml H₂O isotonique (NaCl 8,5 gr/L) ajusté à pH 6,9 stérile, 1 ml de Bouillon PD2

Le bouillon PD2 est obtenu par mélange des solutions A et B décrites ci-après :
Solution A :
   Eau distillée 1L :
      - Soy peptone: 2,0 gr
      - Bacto tryptone: 4,0 gr
      - Disodium succinate : 1,0 gr
      - Trisodium citrate: 1,0 gr
      - K₂HPO₄: 1,5 gr
      - KH₂PO₄ : 1,0 gr
      - Hemin chloride stock solution (0,1% dans (0,05 N NaOH : 0,112 gr/40 ml)) : 10,0 ml
      - MgSO₄.7H₂O : 1,0 gr
      - pH : 6,9
   Autoclaver à 121°C pendant 15 min.
Solution B
   - Bovine serum albumine fraction V (20% w/v) : 10 ml. Stériliser à froid (filtration 0,2 µm).

Mélanger la solution A avec la solution B : lorsque le milieu autoclavé (A) est refroidi à 50°C, ajouter l'albumine stérilisé (B).

*L'agent de biocontrôle* est obtenu par mélange de 1 ml inoculum, 1 ml agent inhibiteur et 1 ml Bouillon PD2, incubé sous agitation (100 rpm) à 26°C pendant 30 minutes.

Les témoins sont obtenus par mélange de 4 gouttes (10 µl)/boîte ; à gauche de la boîte) x5 boîtes et incubation pdt 14 jours 26°c

L'agent de biocontrôle par mélange de 4 gouttes (10 µl)/boîte ; à droite de la boîte) x5 boîtes et incubation pdt 14 jours 26°c

Les boites sont observées au binoculaire x30 et photographiées.

Les photographies sont présentées aux figures 9 à 11

### Exemple 15 : importance du retrait de l'enzyme

Lorsque l'on fabrique les ions recherchés si on maintient l'enzyme dans le mélange, on observe une perte progressive des ions recherchés et après 48h une perte totale des ions recherchés dû à une oxydation enzymatique des ions recherchés comme illustré à la Figure 19 sur laquelle on observe une diminution de l'intensité du signal en fonction du temps de présence de l'enzyme dans la matrice (solution).

### Exemple 16 : Ajout d'enzymes supplémentaires

La présence d'enzymes supplémentaires confère un effet inattendu comme illustré à la Figure 20.

On observe notamment que l'ajout de lactoferrine (>5 mg/L) confère une activité antimicrobienne améliorée par rapport aux solutions sans ajout de lactoferrine lorsque le mélange est dilué 10 fois.

### Exemple 17 : comparaison à l'art antérieur

Non obtention des espèces I₂SCN⁻ ou I(SCN)₂⁻.

Des compositions comprenant un mélange enzymatique avec un ratio KI/KSCN de 1,74 ont été préparées selon les protocoles décrits dans EP1349457.

Des compositions comprenant un mélange enzymatique avec un ratio KI/KSCN de 1,55 ont été préparées selon les protocoles décrits dans WO00/01237.

Les spectres RMN ont été effectués selon les conditions suivantes : un appareil Bruker AMX-500 MHz avec une sonde à large bande de 8 mm a été utilisé. Les spectres ont été obtenus à partir du mélange réactionnel (lactoperoxydase / [¹³C] SCN⁻ / I⁻/ H₂O₂ selon le procédé décrit. L'échantillon consiste en 540 µl de mélange réactionnel, 60 µl D₂O (oxyde de deuterium), 2 µl DSS (acide 4,4-diméthyl-4-silapentane-1-sulfonique). Les échantillons ont été placés dans un tube RMN de 8-mm de long et d'une paroi de 8 pouces. Les spectres ont été recueillis en utilisant les paramètres suivants: largeur de balayage = 15 009, le nombre de points = 32000, le temps d'acquisition = 1.066 s, retard de recyclage de 2s, nombre de balayages = 2000. Les déplacements chimiques (ppm) ont été référencé par rapport au standard d'étalonnage en spectroscopie RMN, le DSS (acide 4,4-diméthyl-4-silapentane-1-sulfonique).

Les Figures 12 à 16 sont les spectres obtenus avec les compositions suivantes :
- Figure 12 : Mélange de 1,86 mM KI + 1,2 mM KSCN (ratio 1,55) + 3,06 mM H₂O₂ dans une matrice aqueuse en présence de lactoperoxydase. Retrait de l'enzyme après 1 minute, 60 minutes, 3 heures, 24 heures ou 48 heures .
- Figure 13 : Mélange de 1,86 mM KI + 1,2 mM KSCN (ratio 1,55) + 3,06 mM H₂O₂ dans une matrice tamponnée acide (tampon citrate 100 mM pH 5) en présence de lactoperoxydase pendant 1 minute, 60 minutes, 3 heures, 24 heures ou 48 heures. Les signaux correspondante au tampon citrate ont été éliminés pour plus de clarté.
- *Figure 14* : Illustration des signaux correspondants au tampon citrate et au SCN⁻.
- *Figure 15* : Mélange de 1,86 mM KI + 1,2 mM KSCN (ratio 1,55) + 3,06 mM H₂O₂ dans une matrice tamponnée neutre (tampon phosphate 100 mM pH 7,4) en présence de lactoperoxydase pendant 1 minute, 60 minutes, 3 heures, 24 heures ou 48 heures.
- Figure 16 : Mélange 5,4 mM KI + 1,2 Mm KSCN + 6,6 Mm H₂O₂ en présence de lactoperoxydase.

On observe le pic correspondant au KS¹³CN quel que soit la matrice utilisée. Il n'y pas de pics à 49-50 ppm. Dans les mélanges décrits dans WO00/01237 ou EP1349457, il n'y a pas de fabrication d'ion I₂SCN⁻ ou I(SCN)₂⁻.

### Démonstration de la présence d'ions hypothiocyanites.

Figure 17 : Mélange de 1,86 mM KI + 1,2 mM KSCN (ratio 1,55) + 3,06 mM H₂O₂ dans une matrice aqueuse en présence de lactoperoxydase. Retrait de l'enzyme après 24 heures.

Figure 18 : Mélange de 1,86 mM KI + 1,2 mM KSCN (ratio 1,55) + 3,06 mM H₂O₂ dans une matrice tamponnée acide (tampon citrate 100 mM pH 5) en présence de lactoperoxydase pendant 3 heures.

Dans les mélanges enzymatiques préparés selon WO00/01237 ou EP1349457, les ions hypothiocyanites sont détectés en faible quantité ainsi que les ions cyanates (OCN⁻), parfaitement reconnaissable grâce à son signal en triplet (Gerritsen et al. 1993) qui correspondent à la dégradation des ions OSCN⁻.

### Exemple 18 : Comparaison des cinétiques des oxydations enzymatiques et chimiques et des activités antimicrobiennes

### • A) Rapidité de la fabrication des ions ions recherché (cinétique enzymatique) ce qui implique une activité antimicrobienne immédiate pour le mélange enzymatique

Cinétique de fabrication des ions recherchés (mesure par oxydation des groupes -SH ou -NH₂)

Des solutions comprenant 5,4 mM KI + 1,2 mM KSCN + 6,6 mM H₂O₂ dans un tampon acétate de sodium 100 mM pH 4,4 +/- LP selon l'invention décrite sont préparées

**Tableau 10**

| **Sans Lactoperoxydase** | | **Avec Lactoperoxydase:** incubation pendant 1 minute puis retrait de la lactoperoxydase | |
|---|---|---|---|
| **Temps (min.)** | **Oxydation -SH (µM/L)** | **Temps (min.)** | **Oxydation -SH (µM/L)** |
| 0 | 4 | 0 | 517 |
| 3 | 49 | 3 | 568 |
| 5 | 74 | 5 | 586 |
| 10 | 133 | 10 | 587 |
| 15 | 168 | 15 | 587 |
| 20 | 183 | 20 | 585 |
| 30 | 197 | 30 | 582 |
| 60 | 203 | 60 | 702 |
| 120 | 235 | 120 | 705 |

**Tableau 11**

| **Sans Lactoperoxydase** | | **Avec Lactoperoxydase** : incubation pendant 1 minute puis retrait de la lactoperoxydase | |
|---|---|---|---|
| **Temps (min.)** | **Oxydation - NH₂ (µM/L)** | **Temps (min.)** | **Oxydation -NH₂ (µM/L)** |
| 1 | 53 | 1 | 2521 |
| 3 | 54 | 3 | 1924 |
| 5 | 107 | 5 | 1426 |
| 10 | 158 | 10 | 1194 |
| 15 | 218 | 15 | 991 |
| 20 | 220 | 20 | 970 |
| 30 | 253 | 30 | 829 |
| 60 | 308 | 60 | 947 |

On constate que la cinétique de formation des ions recherchés est tout à fait différente : la fabrication des ions recherchés avec l'enzyme est instantanée tandis que celle obtenue sans l'enzyme est assez lente, après 1 heure d'incubation environ 1/3 seulement des ions recherchés est obtenu. Ceci a une implication sur l'activité immédiate du mélange actif :
Activité vis-à-vis de *Penicillium expansum*

**Tableau 12**

| **Inhibition *in vitro* vis-à-vis de 1 10⁶ spores/ml de *Pénicillium expansum* du mélange (5,4 mM KI + 1,2 mM KSCN + 6,6 mM H₂O₂) sans Lactoperoxydase (mélange chimique)** | **% d'inhibition à 48h** | **% d'inhibition à 120h** |
|---|---|---|
| 5,4 mM KI + 1,2 mM KSCN + 6,6 mM H₂O₂ eau ville 1/3 | 34 | 4 |
| 5,4 mM KI + 1,2 mM KSCN + 6,6 mM H₂O₂ eau de ville 1/5 | 30 | 33 |
| 5,4 mM KI + 1,2 mM KSCN + 6,6 mM H₂O₂ eau de ville 1/10 | 25 | 32 |
| 5,4 mM KI + 1,2 mM KSCN + 6,6 mM H₂O₂ eau de ville 1/15 | 25 | 7 |
| 5,4 mM KI + 1,2 mM KSCN + 6,6 mM H₂O₂ eau de ville 1/20 | 18 | 3 |
| 5,4 mM KI + 1,2 mM KSCN + 6,6 mM H₂O₂ eau de ville 1/30 | 18 | 29 |
| 5,4 mM KI + 1,2 mM KSCN + 6,6 mM H₂O₂ + eau de ville pH 4,4 1/3 | 17 | 0 |
| 5,4 mM KI + 1,2 mM KSCN + 6,6 mM H₂O₂ + eau de ville pH 4,4 1/5 | 9 | 0 |
| 5,4 mM KI + 1,2 mM KSCN + 6,6 mM H₂O₂ + eau de ville pH 4,4 1/10 | 14 | 2 |
| 5,4 mM KI + 1,2 mM KSCN + 6,6 mM H₂O₂ + eau de ville pH 4,4 1/15 | 16 | 27 |
| 5,4 mM KI + 1,2 mM KSCN + 6,6 mM H₂O₂ + eau de ville pH 4,4 1/20 | 14 | 22 |
| 5,4 mM KI + 1,2 mM KSCN + 6,6 mM H₂O₂ + eau de ville pH 4,4 1/30 | 15 | 23 |
| 5,4 mM KI + 1,2 mM KSCN + 6,6 mM H₂O₂ + tp acétat 0,1M pH 4,5 1/3 | 16 | 21 |
| 5,4 mM KI + 1,2 mM KSCN + 6,6 mM H₂O₂ + tp acétat 0,1M pH 4,5 1/5 | 31 | 17 |
| 5,4 mM KI + 1,2 mM KSCN + 6,6 mM H₂O₂ + tp acétat 0,1M pH 4,5 1/10 | 21 | 21 |
| 5,4 mM KI + 1,2 mM KSCN + 6,6 mM H₂O₂ + tp acétat 0,1M pH 4,5 1/15 | 26 | 37 |
| 5,4 mM KI + 1,2 mM KSCN + 6,6 mM H₂O₂ + tp acétat 0,1M pH 4,5 1/20 | 29 | 42 |
| 5,4 mM KI + 1,2 mM KSCN + 6,6 mM H₂O₂ + tp acétat 0,1M pH 4,5 1/30 | 35 | 35 |

**Tableau 13**

| **Inhibition *in vitro* vis-à-vis de 1 10⁶ spores/ml de *Pénicillium expansum* du mélange (5,4 mM KI + 1,2 mM KSCN + 6,6 mM H₂O₂) avec Lactoperoxydase pendant 1 minute (mélange enzymatique)** | **% d'inhibition à 48h** | **% d'inhibition à 120h** |
|---|---|---|
| B1 Tp acétate 0,5M pH 4,5 1/3 | 96 | 97 |
| B1 Tp acétate 0,5M pH 4,5 1/5 | 96 | 97 |
| B1 Tp acétate 0,5M pH 4,5 1/10 | 96 | 98 |
| B1 Tp acétate 0,5M pH 4,5 1/15 | 96 | 98 |
| B1 Tp acétate 0,5M pH 4,5 1/20 | 97 | 98 |
| B1 Tp acétate 0,5M pH 4,5 1/30 | 95 | 60 |
| B1 Tp acétate 0,5M pH 4,5 1/50 | 81 | 37 |
| B1 Tp acétate 0,1M pH 4,5 1/3 | 94 | 96 |
| B1 Tp acétate 0,1M pH 4,5 1/5 | 96 | 97 |
| B1 Tp acétate 0,1M pH 4,5 1/10 | 95 | 97 |
| B1 Tp acétate 0,1M pH 4,5 1/15 | 97 | 98 |
| B1 Tp acétate 0,1M pH 4,5 1/20 | 70 | 36 |
| B1 Tp acétate 0,1M pH 4,5 1/30 | 53 | 22 |
| B1 Tp acétate 0,1M pH 4,5 1/50 | 40 | 4 |
| B1 Tp acétate 0,01M pH 4,5 1/3 | 93 | 96 |
| B1 Tp acétate 0,01M pH 4,5 1/5 | 94 | 96 |
| B1 Tp acétate 0,01M pH 4,5 1/10 | 94 | 97 |
| B1 Tp acétate 0.01M pH 4,5 1/15 | 81 | 99 |
| B1 Tp acétate 0,01M pH 4,5 1/20 | 53 | 0 |
| B1 Tp acétate 0,01M pH 4,5 1/30 | 19 | 0 |
| B1 Tp acétate 0,01M pH 4,5 1/50 | 6 | 0 |
| B1 Tp acétate 0,001M pH 4,5 1/3 | 100 | 100 |
| B1 Tp acétate 0,001M pH 4,5 1/5 | 97 | 99 |
| B1 Tp acétate 0,001M pH 4,5 1/10 | 96 | 99 |
| B1 Tp acétate 0.001M pH 4,5 1/15 | 95 | 99 |
| B1 Tp acétate 0.001M pH 4,5 1/20 | 29 | 0 |
| B1 Tp acétate 0,001M pH 4,5 1/30 | 8 | 0 |
| B1 Tp acétate 0,001M pH 4,5 1/50 | 13 | 0 |

On constate que le mélange chimique (temps d'incubation des réactifs : 1 minute) n'est pas efficace pour inhiber la croissance *in vitro* de *Penicillium expansum.*

A contrario le mélange enzymatique de (5,4 mM KI + 1,2 mM KSCN + 6,6 mM H₂O₂) + lactoperoxydase pendant 1 minute puis retrait de l'enzyme est efficace jusqu'à la dilution 1/30 pour le tampon 500 mM, jusqu'à la dilution 1/15 avec le tampon 100 mM, 10 mM et 1 mM :

La méthode de préparation du mélange actif contenant les ions recherchés, c'est-à-dire chimique ou enzymatique, a une implication sur l'activité antimicrobienne immédiate du mélange. Le mélange enzymatique a une efficacité antimicrobienne immédiate (présente dès 1 minute d'incubation), alors que cette activité antimicrobienne est absente après 1 minute d'incubation des substrats dans le mélange chimique.

### Exemple 19 : Activité antimicrobienne vis-à-vis d' E. coli d'une composition immobilisée sur un tissu et lyophilisée

Une composition selon l'invention a été préparée par mise en présence de 5,4 mM d'iodure de potassium (KI) de 1,2 mM de thiocyanate de potassium (KSCN) de 6,6 mM de peroxyde d'hydrogène (H₂O₂) en présence de 50 mg/L de lactoperoxydase (LP)(1000 unités ABTS par mg) dans un tampon citrate de sodium 100 mM pH 6,2 (figure 23b) ou un tampon phosphate 100 mM pH 7,4 (figure 23c) ou un tampon citrate de sodium 100 mM pH 6,2 et lyophilisé après préparation et reconstitué dans de l'eau (23d). Ces compositions ont été immobilisées sur un tissu et l'activité antibactérienne de ces tissus imbibés de ces compositions a été testée vis-à-vis d'*E. coli* (figure 23b, 23c, et 23d).

Il est visible que la composition lyophilisée conserve une action bactéricide équivalente aux autres compositions non lyophilisées (figure 23b et 23c versus figure 23d). Le tissu du « contrôle » a été imbibé d'eau stérile (figure 23a).

Les figures 23 illustrent l'action qu'a la composition selon l'invention sur *E. coli* (10⁹ cfu/ml).

Il est apparent que après 24h d'incubation à 37°C de 100 µl d'*E. coli* à 10⁹ cfu/ml sur un milieu de culture sur boîte de petri, la composition immobilisée sur un tissu empêche le développement de la bactérie (halo apparent)

## Revendications

1. Composition stable obtenue par oxydation enzymatique d'un mélange iodure thiocyanate, comprenant au moins un ion choisi dans le groupe constitué des ions I₂SCN⁻ et des ions I(SCN)₂⁻, ladite composition étant exempte d'ions hypothiocyanite (OSCN⁻).

2. Composition stable selon la revendication 1, **caractérisée en ce qu'**elle comprend en outre du thiocyanate d'iode ISCN.

3. Composition stable selon l'une quelconque des revendications 1 ou 2, **caractérisée en ce qu'**elle comprend en outre au moins un composé choisi dans le groupe constitué de la lactoferrine, du lysozyme, des immunoglobines, des facteurs de croissance et leurs mélanges.

4. Procédé de fabrication d'une composition stable selon l'une quelconque des revendications précédentes, comprenant :
- une étape A de préparation d'un milieu réactionnel comprenant au moins deux substrats, au moins un oxydant, et un catalyseur, la mise en présence dudit catalyseur et dudit oxydant étant subordonnée à la mise en présence desdits deux substrats ;
- une étape B de réaction débutant à la mise en présence dudit oxydant et dudit catalyseur ;
- une étape C de retrait dudit catalyseur, et de récupération d'une composition selon l'invention comprenant au moins des ions I₂SCN⁻ et/ou des ions I(SCN)₂⁻ ;
lesdits substrats étant des ions iodure (I-) et thiocyanate (SCN-),
ledit oxydant étant un système générateur de peroxyde d'hydrogène (H₂O₂) et/ou du peroxyde d'hydrogène, le catalyseur étant au moins une peroxydase,
ledit procédé étant **caractérisé en ce que** ladite étape de réaction a une durée comprise entre 30 et 1800 secondes et **en ce qu'**il ne donne pas lieu à la formation d'ion hypothiocyanite (OSCN⁻).

5. Procédé selon la revendication 4, **caractérisé en ce qu'**il comprend en outre une étape de lyophilisation de la composition selon l'invention à l'issue de laquelle un lyophilisat est obtenu, ledit lyophilisat permettant, lors d'une remise en solution, la reconstitution de ladite composition qui comprend au moins des ions I₂SCN⁻ et/ou des ions I(SCN)₂⁻, et est exempte d'ion hypothiocyanite (OSCN⁻).

6. Procédé selon l'une quelconque des revendications 4 à 5, **caractérisé en ce que** le rapport entre la concentration molaire en ion thiocyanate (SCN⁻) et la concentration molaire en ion iodure (I⁻) est strictement supérieur à 1.

7. Procédé selon l'une quelconque des revendications 4 à 6, **caractérisé en ce que** le pH de la solution est compris entre 4 et 8.

8. Procédé selon l'une quelconque des revendications 4 à 7, **caractérisé en ce que** le temps de mise en contact est compris entre 30 et 200 secondes.

9. Procédé selon l'une quelconque des revendications 4 à 8, caractérisé que la peroxydase est choisie dans le groupe constitué par la lactoperoxydase (LP), la peroxydase thyroïdienne (TPO), la myéloperoxydase (MPO), la peroxydase salivaire (SPO) et la peroxydase éosinophile (EPO).

10. Procédé selon l'une quelconque des revendications 4 à 9, **caractérisé en ce que** la peroxydase est la lactoperoxydase (LP).

11. Procédé selon l'une quelconque des revendications 4 à 10, **caractérisé en ce que** ladite peroxydase a une concentration comprise 1mg/L et 500mg/L.

12. Procédé selon l'une quelconque des revendications 4 à 11, **caractérisé en ce que** l'ion thiocyanate (SCN⁻) est présent à une concentration molaire comprise entre 0,1 mM et 1 M.

13. Procédé selon l'une quelconque des revendications 4 à 12, **caractérisé en ce que** l'ion iodure (I-) est présent à une concentration molaire comprise entre 0,1 mM et 1 M.

## Patentansprüche

1. Stabile Zusammensetzung erhalten durch enzymatische Oxidation einer Iodidthiocyanat-Mischung, umfassend wenigstens ein Ion, das ausgewählt ist aus der Gruppe bestehend aus I₂SCN⁻-Ionen und I(SCN)₂⁻-Ionen, wobei die Zusammensetzung frei von Hypothiocyanit (OSCN⁻)-Ionen ist.

2. Stabile Zusammensetzung nach Anspruch 1, **dadurch gekennzeichnet, dass** sie weiterhin Iodthiocyanat ISCN umfasst.

3. Stabile Zusammensetzung nach einem der Ansprüche 1 bis 2, **dadurch gekennzeichnet, dass** sie weiterhin wenigstens eine Verbindung umfasst, die ausgewählt ist aus der Gruppe bestehend aus Lactoferrin, Lysozym, Immunglobulinen, Wachstumsfaktoren und Mischungen davon.

4. Verfahren zur Herstellung einer stabilen Zusammensetzung nach einem der vorangehenden Ansprüche, umfassend
- einen Schritt A des Herstellens eines Reaktionsmediums wenigstens umfassend zwei Substrate, wenigstens ein Oxidationsmittel und einen Katalysator, wobei das Zusammenbringen des Katalysators und des Oxidationsmittel abhängig ist von dem Zusammenbringen der zwei Substrate,
- einen Reaktionsschritt B beginnend mit dem Zusammenbringen des Oxidationsmittels und des Katalysators,
- einen Schritt C des Entfernens des Katalysators und der Rückgewinnung einer Zusammensetzung gemäß der Erfindung umfassend wenigstens I₂SCN⁻-Ionen und/oder I(SCN)₂⁻-Ionen;
wobei
die Substrate Iodid (I⁻)-Ionen und Thiocyanat (SCN⁻)-Ionen sind, das Oxidationsmittel ein Wasserstoffperoxid (H₂O₂) erzeugendes System und/oder Wasserstoffperoxid ist, der Katalysator wenigstens eine Peroxidase ist,
das Verfahren **dadurch gekennzeichnet ist, dass** der Reaktionsschritt von 30 bis 1.800 Sekunden dauert und nicht zur Bildung eines Hypothiocyanit (OSCN⁻)-Ions führt.

5. Verfahren nach Anspruch 4, **dadurch gekennzeichnet, dass** es weiterhin einen Lyophilisierungsschritt der erfindungsgemäßen Zusammensetzung umfasst, an dessen Ende ein Lyophilisat erhalten wird, wobei das Lyophilisat, während erneuter Überführung in eine Lösung, die Rekonstitution der Zusammensetzung erlaubt, die wenigstens I₂SCN⁻-Ionen und/oder I(SCN)₂⁻ -Ionen umfasst und frei von einem Hypothiocyanit (OSCN⁻)-Ion ist.

6. Verfahren nach einem der Ansprüche 4 bis 5, **dadurch gekennzeichnet, dass** das Verhältnis der molaren Konzentration von Thiocyanat (SCN⁻)-Ion und der molaren Konzentration von Iodid (I⁻)-Ion unbedingt größer als 1 ist.

7. Verfahren nach einem der Ansprüche 4 bis 6, **dadurch gekennzeichnet, dass** der pH der Lösung zwischen 4 und 8 beträgt.

8. Verfahren nach einem der Ansprüche 4 bis 7, **dadurch gekennzeichnet, dass** die Kontaktdauer zwischen 30 und 200 Sekunden beträgt.

9. Verfahren nach einem der Ansprüche 4 bis 8, **dadurch gekennzeichnet, dass** die Peroxidase ausgewählt ist aus der Gruppe bestehend aus Lactoperoxidase (LP), Schilddrüsenperoxidase (TPO), Myeloperoxidase (MPO), Speichelperoxidase (SPO) und Eosinophilenperoxidase (EPO).

10. Verfahren nach einem der Ansprüche 4 bis 9, **dadurch gekennzeichnet, dass** die Peroxidase Lactoperoxidase (LP) ist.

11. Verfahren nach einem der Ansprüche 4 bis 10, **dadurch gekennzeichnet, dass** die Peroxidase eine Konzentration von 1 mg/L bis 500 mg/L aufweist.

12. Verfahren nach einem der Ansprüche 4 bis 11, **dadurch gekennzeichnet, dass** das Thiocyanat (SCN⁻)-Ion in einer molaren Konzentration von 0,1 mM bis 1 M vorliegt.

13. Verfahren nach einem der Ansprüche 4 bis 12, **dadurch gekennzeichnet, dass** das Iodid (I⁻)-Ion in einer molaren Konzentration von 0,1 mM bis 1 M vorliegt.

## Claims

1. A stable composition obtained by enzymatic oxidation of a iodide thiocyanate mixture, comprising at least one ion selected from the group consisting of the I2SCN- ions and the I(SCN)2- ions, said composition being free of hypothiocyanite ions (OSCN-).

2. The stable composition according to claim 1, wherein it further comprises iodine thiocyanate ISCN.

3. The stable composition according to claim 1 or claim 2, wherein it further comprises at least one compound selected from the group consisting of lactoferrin, lysozyme, immunoglobulins, growth factors and mixtures thereof.

4. A method for manufacturing a stable composition according to any one of the preceding claims, comprising:
- a step A of preparation of a reaction medium comprising at least two substrates, at least one oxidizing agent, and a catalyst, the bringing together of said catalyst and said oxidizing agent being contingent upon the bringing together of said two substrates;
- a reaction step B starting with the bringing together of said oxidizing agent and said catalyst;
- a step C of removal of said catalyst, and of recovery of a composition according to the invention comprising at least one of the I₂SCN⁻ ions and/or of the ions I(SCN)₂⁻ ions;
said substrates being iodide (I-) and thiocyanate (SCN-) ions,
said oxidizing agent being a hydrogen peroxide (H₂O₂) generating system and/or hydrogen peroxide, the catalyst being at least one peroxidase,
said method being **characterized in that** said reaction step has a duration from 30 to 1800 seconds and **in that** it does not give rise to the formation of hypothiocyanite ion (OSCN⁻).

5. The method according to claim 4, wherein it further comprises a step of lyophilization of the composition according to the invention at the end of which a lyophilisate is obtained, said lyophilisate enabling, during a redissolution, the reconstitution of said composition which includes at least one of the I₂SCN⁻ ions and/or of the I(SCN)₂⁻ ions and is free of hypothiocyanite ion (OSCN⁻).

6. The method according to any one of claims 4 to 5, wherein the ratio between the molar concentration of thiocyanate ion (SCN⁻) and the molar concentration of iodide ion (I⁻) is strictly greater than 1.

7. The method according to any one of claims 4 to 6, wherein the pH of the solution is from 4 to 8.

8. The method according to any one of claims 4 to 7, wherein the contact time, at step 5, is from 30 to 200 seconds.

9. The method according to any one of claims 4 to 8, wherein the peroxidase is selected from the group consisting of the lactoperoxidase (LP), the thyroid peroxidase (TPO), the myeloperoxidase (MPO), the salivary peroxidase (SPO) and the eosinophil peroxidase (EPO).

10. The method according to any one of claims 4 to 9, wherein the peroxidase is the lactoperoxidase (LP).

11. The method according to any one of claims 4 to 10, wherein said peroxidase has a concentration from 1 mg/L to 500 mg/L.

12. The method according to any one of claims 4 to 11, wherein the thiocyanate ion (SCN⁻) is present at a molar concentration from 0.1 mM to 1 M.

13. The method according to any one of claims 4 to 12, wherein the iodide ion (I⁻) is present at a molar concentration from 0.1 mM to 1 M.
